# EUROPEAN PATENT SPECIFICATION

(11) **EP 2 632 440 B1**
(45) Date of publication and mention of the grant of the patent: **12.06.2019**
(21) Application number: 11837163.2
(22) Date of filing: 28.10.2011
(51) Int. Cl.: A61K 9/24, A61K 9/00, A61K 47/10

(54) **PROCESS FOR ANALYZING AND ESTABLISHING DOSAGE SIZE IN AN INGESTIBLE FILM**
VERFAHREN ZUR ANALYSE UND FESTLEGUNG DER DOSIERUNGSMENGE EINES EINNEHMBAREN FILMS
PROCÉDÉ D'ANALYSE ET DE DÉTERMINATION DE LA TAILLE D'UNE DOSE DANS UN FILM À INGÉRER

(30) Priority: 29.10.2010 US 915849
(43) Date of publication of application: 04.09.2013
(73) Proprietor: Aquestive Therapeutics, Inc., Warren, NJ 07059 (US)
(72) Inventor: MYERS, Garry, Kingsport TN 37660 (US); BOGUE, Beuford, A., New Carlisle IN 46552 (US); HARIHARAN, Madhu, Munster IN 46321 (US)
(74) Representative: EP&C
(86) International application number: PCT/US2011/058296
(87) International publication number: WO 2012/058545

(56) References cited:
- EP-A1- 2 196 198
- WO-A2-2007/030754
- US-A- 4 332 789
- US-A- 4 332 789
- US-A1- 2004 076 799
- US-A1- 2004 076 799
- US-A1- 2008 044 454
- US-A1- 2008 044 454
- US-A1- 2008 260 805
- US-A1- 2011 208 348
- US-A1- 2012 107 402
- DIXIT R P ET AL: "Oral strip technology: Overview and future potential", JOURNAL OF CONTROLLED RELEASE, ELSEVIER, AMSTERDAM, NL, vol. 139, no. 2, 15 October 2009 (2009-10-15), pages 94-107, XP026601268, ISSN: 0168-3659, DOI: 10.1016/J.JCONREL.2009.06.014 [retrieved on 2009-06-24]

## Description

### FIELD OF THE INVENTION

The present invention relates to a process of analyzing and establishing dosage size in ingestible films that contain an active ingredient that is distributed throughout the film.

### BACKGROUND OF THE RELATED TECHNOLOGY

Ingestible films that dissolve in water and are edible are known in the art for use in the delivery of active ingredients. Such films dissolve in the oral cavity, releasing the active ingredient. In the preparation of such films, the active ingredients are dispersed in the film material. Although the active ingredient is generally uniformly dispersed in the film, variation in the dispersion of the active ingredient can result in the administration of an amount of the active ingredient which is less than or greater than the desired dosage.

Examination of films made in accordance with conventional air drying methods reveal that such films suffer from the aggregation or conglomeration of particles, i.e., self-aggregation, making them inherently non-uniform. This result can be attributed to process parameters, which although not usually disclosed likely include the use of relatively long drying times, thereby facilitating intermolecular attractive forces, convection forces, air flow and the like to form such agglomeration.

The formation of agglomerates randomly distributes the film components and any active present as well. When large dosages are involved, a small change in the dimensions of the film would lead to a large difference in the amount of active per film. If such films were to include low dosages of active, it is possible that portions of the film may be substantially devoid of any active. Since sheets of film are usually cut into unit doses, certain doses may therefore be devoid of or contain an insufficient amount of active for the recommended treatment. Failure to achieve a high degree of accuracy with respect to the amount of active ingredient in the cut film can be harmful to the patient. For this reason, dosage forms formed by processes such as Fuchs, would not likely meet the stringent standards of governmental or regulatory agencies, such as the U.S. Federal Drug Administration ("FDA"), relating to the variation of active in dosage forms. Currently, as required by various world regulatory authorities, dosage forms may not vary more than 10% in the amount of active present. When applied to dosage units based on films, this virtually mandates that uniformity in the film be present.

Furthermore, these methods employ the use the conventional time-consuming drying methods such as a high-temperature air-bath using a drying oven, drying tunnel, vacuum drier, or other such drying equipment. The long length of drying time aids in promoting the aggregation of the active and other adjuvant, notwithstanding the use of viscosity modifiers. Such processes also run the risk of exposing the active, i.e., a drug, or biological, or other components to prolonged exposure to moisture and elevated temperatures, which may render it ineffective or even harmful.

In addition to the concerns associated with degradation of an active during extended exposure to moisture, the conventional drying methods themselves are unable to provide uniform films. The length of heat exposure during conventional processing, often referred to as the "heat history", and the manner in which such heat is applied, have a direct effect on the formation and morphology of the resultant film product. Uniformity is particularly difficult to achieve via conventional drying methods where a relatively thicker film, which is well-suited for the incorporation of a drug active, is desired. Thicker uniform films are more difficult to achieve because the surfaces of the film and the inner portions of the film do not experience the same external conditions simultaneously during drying. Thus, observation of relatively thick films made from such conventional processing shows a non-uniform structure caused by convection and intermolecular forces and requires greater than 10% moisture to remain flexible. The amount of free moisture can often interfere over time with the drug leading to potency issues and therefore inconsistency in the final product.

Conventional drying methods generally include the use of forced hot air using a drying oven, drying tunnel, and the like. The difficulty in achieving a uniform film is directly related to the rheological properties and the process of water evaporation in the film-forming composition. When the surface of an aqueous polymer solution is contacted with a high temperature air current, such as a film-forming composition passing through a hot air oven, the surface water is immediately evaporated forming a polymer film or skin on the surface. This seals the remainder of the aqueous film-forming composition beneath the surface, forming a barrier through which the remaining water must force itself as it is evaporated in order to achieve a dried film. As the temperature outside the film continues to increase, water vapor pressure builds up under the surface of the film, stretching the surface of the film, and ultimately ripping the film surface open allowing the water vapor to escape. As soon as the water vapor has escaped, the polymer film surface reforms, and this process is repeated, until the film is completely dried. The result of the repeated destruction and reformation of the film surface is observed as a "ripple effect" which produces an uneven, and therefore non-uniform film. Frequently, depending on the polymer, a surface will seal so tightly that the remaining water is difficult to remove, leading to very long drying times, higher temperatures, and higher energy costs.

Other factors, such as mixing techniques, also play a role in the manufacture of a pharmaceutical film suitable for commercialization and regulatory approval. Air can be trapped in the composition during the mixing process or later during the film making process, which can leave voids in the film product as the moisture evaporates during the drying stage. The film frequently collapse around the voids resulting in an uneven film surface and therefore, non-uniformity of the final film product. Uniformity is still affected even if the voids in the film caused by air bubbles do not collapse. This situation also provides a non-uniform film in that the spaces, which are not uniformly distributed, are occupying area that would otherwise be occupied by the film composition. None of the above-mentioned patents either addresses or proposes a solution to the problems caused by air that has been introduced to the film. EP-2196198 teaches a method for manufacture of edible film comprising active agent(s) in uniform distribution.

All dose forms are either manufactured and dosed according to weight for solid forms like capsules, tablets and milliliters of liquid for liquid forms. The conventional method of preparing dosage forms result in variance and loss of volatiles during the drying stage. In order to correct the variance and make sure that the active is uniform and in compliance with the intended dosage amount, multiple batched are required to be run which can be very time consuming and costly.

Therefore, there is a need in the art for improved methods for analyzing and establishing a proper dosage size in ingestible films for providing a more precise dosage delivery of an active ingredient.

### SUMMARY OF THE INVENTION

The present invention is directed to a process for producing an ingestible film comprising an active component, in which the ingestible film has a predetermined dosage for an active component. The process includes the steps of: preparing an ingestible film that includes an active component; analyzing the film to determine the amount of the active component per unit weight of the film and based on the amount of the component per unit weight of the film; determining the dimensions and/or weight of the film necessary to deliver the predetermined dosage for the active component; and adjusting the dimensions and/or weight of the film into the dimensions and/or weight determined.

The present invention is also directed to a process for producing an ingestible film comprising an active component, said ingestible film having a predetermined dosage for said active component. The process includes combining a polymer component, water and an active component to form a matrix with a uniform distribution of said components; forming a film from said matrix; providing a conveyor surface having top and bottom sides; feeding said film onto said top side of said surface; analyzing said film to determine the amount of said active component per unit weight of said film and based on said amount of said component per unit weight of said film determining the dimension of the film necessary to deliver said predetermined dosage for said active component; and adjusting said film into the dimension determined in the previous step.

The present invention is also directed to a process for producing an ingestible film dosage unit comprising an active component, said ingestible film dosage unit having a predetermined % dosage for said active component, said film dosage unit having a predetermined width and length. The process includes preparing two or more films comprising the steps of: preparing a first continuous film comprising a polymer component and an active component uniformly dispersed in said polymer component; said first continuous film having a constant width and length effective to produce said predetermined width and length of said ingestible film dosage unit when cut, and said first continuous film has a first thickness, and preparing a second continuous film comprising a polymer component and an active component uniformly dispersed in said polymer component; said second continuous film having the constant width and length of said first film and having a second thickness; analyzing said two films to determine the thickness required for preparing said ingestible film dosage unit having the predetermined % dosage for said active component when said a continuous film is cut into a film dosage unit having said predetermined length and width; preparing a continuous film having said thickness determined in previous step; cutting said continuous film to produce film dosage units having said thickness determined in previous step and said predetermined length and width.

The present invention is also directed to a process for producing an ingestible film dosage unit comprising an active component, said ingestible film dosage unit having a predetermined % dosage for said active component. The process includes preparing a continuous film comprising a polymer component and an active component uniformly dispersed in said polymer component; drying said film; analyzing said dried film off line to determine the % of active per unit weight of said film; using information obtained in previous step to determine the width and length of the film effective to produce a film having said predetermined dosage; cutting said film to the width and length determined in the previous step to produce ingestible film dosage units.

### BRIEF DESCRIPTION OF THE FIGURES

Figure 1 depicts a linear correlation between the assays at different strip weights for the active drug and the piece weight.
Figure 2 is a graphical depiction of the results of the assay values shown in Table 5 and are plotted against the piece weight.
Figure 3 depicts the absorbance spectra for Example 4.
Figure 4 depicts the second derivative absorbance spectra for Example 4.
Figures 5-8 depict the actual measured values plotted along with the control limits in the 4 plots, of Table 9 of Example 5.
Figures 9-12 depict the actual measured values plotted along with the control limits in the 4 plots, of Table 11 of Example 5.
Figure 13 depicts the actual measured values are plotted along with the control limits as shown in Table 13 of Example 5.
Figure 14 depicts the NDC values vs. weight data in order to demonstrate that a calculation can be made to show if it is necessary to adjust the film dimensions

### DETAILED DESCRIPTION OF THE INVENTION

In describing the present invention, the following terms will be employed, and are intended to be defined as indicated below.

As used herein, the terms "pharmaceutical", "medicament", "drug" and "active" may be used interchangeably, and refer to a substance or composition useful for the prevention or treatment of a condition. The terms may include pharmaceuticals, neutraceuticals, cosmetic agents, biologic agents, bioeffective substances, and the like.

The term "film" includes delivery systems of any thickness, including films, sheets, discs, wafers, and the like, in any shape, including rectangular, square, or other desired shape. The film may be in the form of a continuous roll of film or may be sized to a desired length and width. The films described herein may be any desired thickness and size suitable for the intended use. For example, a film of the present invention may be sized such that it may be placed into the oral cavity of the user or adhered to mucosal or organ tissue. For example, some films may have a relatively thin thickness of from about 0.1 to about 10 mils, while others may have a somewhat thicker thickness of from about 10 to about 30 mils. For some films, the thickness may be even larger, i.e., greater than about 30 mils. It will be understood, of course, that the thickness of the film may be limited due to the formulation used, and thicker films may require longer drying times or different manufacturing techniques.

Further, thicker films may desirably be formed through lamination of thinner films. In addition, the term "film" includes single-layer compositions as well as multi-layer compositions, such as laminated films, coatings on films and the like. For example, two or more films may be separately formed and then laminated together using, for example, heat and/or solvent to form a thicker film. Additionally, multiple layers of film may be made by coating a first film with additional film layers, without necessarily the need for lamination steps. Multiple layers of film may be added to form structures of various thicknesses and also to allow for different functions and properties of the different layers. The composition, regardless of thickness, maintains a uniform distribution of components through the application of controlled drying of the film to provide a final film which in its dried form has uniformity of the active throughout the film, as described herein. The films of the present invention will not vary in active content of more than 10% by weight in any given weight of film. For example, unit doses of equal or random sizes will contain substantially the same amount by weight of active, with no more than a variation of 10% by weight between the doses. Film structures of the present invention may also include a pouch or region of drug between two films.

For the purposes of the present invention the term non-self-aggregating uniform heterogeneity refers to the ability of the films of the present invention, which are formed from one or more components in addition to a polar solvent, to provide a substantially reduced occurrence of, i.e. little or no, aggregation or conglomeration of components within the film as is normally experienced when films are formed by conventional drying methods such as a high-temperature air-bath using a drying oven, drying tunnel, vacuum drier, or other such drying equipment. The term heterogeneity, as used in the present invention, includes films that will incorporate a single component, such as a polymer, as well as combinations of components, such as a polymer and an active. Uniform heterogeneity includes the substantial absence of aggregates or conglomerates as is common in conventional mixing and heat drying methods used to form films.

Uniformity of components throughout the film, i.e. uniformity of content, is beneficial in administering an accurate and effective dose to a user. Various methods of forming uniform films, as well as various additives and fillers, may be used, including those methods and materials described in U.S. Patent Nos. 7,425,292, 7,357,891, and 7,666,337. In some particularly desirable embodiments, the amount of active, or the amount of active per se, per unit weight does not vary more than about 10%, as discussed above. Thus a large sheet of film may be made and equally sized dosage units cut therefrom and the amount of active or active per se in each dosage unit will not vary more than 10% by weight between units.

Furthermore, the films of the present invention have a substantially uniform thickness, which is also not provided by the use of conventional drying methods used for drying water-based polymer systems. The absence of a uniform thickness detrimentally affects uniformity of component distribution throughout the area of a given film.

The present invention is directed to a method by which one would analyze the film continuously with online analyzers or by statistical sampling.

In accordance with an embodiment of the present invention, a process for producing an ingestible film including an active component in which the amount of the active component in the film is controlled by analyzing the film prior to packaging to determine the amount of the active ingredient.

In one embodiment, the film is analyzed to determine the amount of the active component per unit of weight of the film.

### Preparing a Film

As discussed herein, a flowable film-forming matrix is prepared to be uniform in content in accordance with the teachings of the present invention. Uniformity of content is desirably maintained as the flowable matrix is formed into a film and dried. The drying process of the present invention may use several factors to produce uniformity within the film, while maintaining the active component at a safe temperature, *i.e*., at temperatures and/or conditions where the active won't substantially degrade, or become less potent or substantially inactive. First, the films of the present invention have an extremely short heat history, usually only on the order of minutes, so that total temperature exposure is minimized to the extent possible. The films are controllably dried to prevent aggregation and migration of components, as well as preventing heat build up within. The films may be dried from the bottom or a combination of top and bottom drying. Desirably, the top surface of the wet film is not dried in a manner which causes skinning prior to drying the thickness of the film to the desired final water content level, which as will be described later herein, is about 10% by weight or less of the total film composition.

In any drying method, however, it is desirable to rapidly form an active-immobilizing visco-elastic mass of the film within the first ten (10) minutes to fifteen (15) minutes of drying, more desirably within the first four (4) to six (6) minutes of drying and most desirably within the first four (4) minutes of drying to create a uniform distribution of said active by locking-in or substantially preventing migration of said active. Due to the short heat exposure and evaporative cooling, the film components such as drug, sensitive biologicals or volatile actives remain unaffected by high temperatures during the drying process, and the active is maintained in a non-aggregated fashion. In contrast, skinning on the top surface traps liquid carrier molecules of increased energy within the film, thereby causing the temperature within the film to rise and exposing active components to high, potentially deleterious temperatures.

Second, thermal mixing occurs within the film due to controlled drying and absence of surface skinning. Thermal mixing occurs via convection currents in the film. As heat is applied to the bottom of the film, the liquid near the bottom increases in temperature, expands, and becomes less dense. As such, this hotter liquid rises and cooler liquid takes its place. While rising, the hotter liquid mixes with the cooler liquid and shares thermal energy with it, *i.e*., transfers heat. As the cycle repeats, thermal energy is spread throughout the film.

Robust thermal mixing achieved by the controlled drying process of the present invention produces uniform heat diffusion throughout the film. In the absence of such thermal mixing, "hot spots" may develop. Pockets of heat in the film result in the formation of particle aggregates or danger areas within the film and subsequent non-uniformity. The formation of such aggregates or agglomerations is undesirable because it leads to non-uniform films in which the active may be randomly distributed. Such uneven distribution may lead to large differences in the amount of active per film unit, dosage or weight, which is problematic from a potency, safety and efficacy perspective.

Furthermore, thermal mixing helps to maintain a lower overall temperature inside the film. Although the film surfaces may be exposed to a temperature above that at which the active component degrades, the film interior may not reach this temperature. Due to this temperature differential, the active does not degrade.

For instance, the films of the present invention desirably are dried for ten (10) minutes or less. Drying the films at 80°C for ten (10) minutes produces a temperature differential between the atmosphere and the film matrix of about 5°C. This means that after ten (10) minutes of drying, the temperature of the inside of the film is 5°C less than the outside exposure temperature. In many cases, however, drying times of less than ten (10) minutes are sufficient, such as four (4) to six (6) minutes. Drying for four (4) minutes may be accompanied by a temperature differential of about 30°C, and drying for six (6) minutes may be accompanied by a differential of about 25°C. Due to such large temperature differentials, the films may be dried at efficient, high air temperatures without causing heat sensitive actives to degrade, and without causing the matrix to reach a temperature where the active becomes substantially unstable, substantially degrades or becomes less active.

After mechanical mixing, the film may be placed on a conveyor for continued thermal mixing during the drying process. At the outset of the drying process, the film preferably is heated from the bottom as it travels via conveyor. Heat may be supplied to the film by a heating mechanism, such as, but not limited to, a dryer. As the film is heated, the liquid carrier, or volatile, begins to evaporate. Thermal mixing also initiates as hotter liquid rises and cooler liquid takes its place. Because no skin forms on the top surface of the film, the volatile liquid continues to evaporate and thermal mixing continues to distribute thermal energy throughout the film. Once a sufficient amount of the volatile liquid has evaporated, thermal mixing has produced uniform heat diffusion throughout the film. The components desirably are locked into a uniform distribution throughout the film. It may be desired to form a visco-elastic solid rapidly, for example within the first ten (10) minutes or less, desirably within the first six (6) minutes or less, and most desirably within the first 0.5 minutes to four (4) minutes. Although minor amounts of liquid carrier, i.e., water, may remain subsequent to formation of the visco-elastic film, the film may be dried further without affecting the desired heterogeneity of the film, if desired. Further drying forms the final film, by desirably removing solvent from the visco-elastic solid such that less than ten percent (10%) of solvent remains, and more desirably less than eight percent (8%) of solvent remains, and most desirably less than six percent (6%) of the solvent remains in the final film.

While the air temperatures for drying may be about 50°C to about 160°C, the temperatures of the film matrix are generally less than the boiling temperature of the solvent in the matrix, desirably about 100°C or less, and more desirably about 90°C or less and most desirably about 80°C. In other words, the air temperatures used for drying may optionally be greater than the actual temperatures which the matrix experiences.

Furthermore, an active may be added to the film-forming composition or material after the composition or material is cast into a film. For example, an active may be added to the film prior to the drying of the film. An active may be controllably metered to the film and disposed onto the film through a suitable technique, such as through the use of a doctor blade, which is a device which marginally or softly touches the surface of the film and controllably disposes the particles onto the film surface. Other suitable, but non-limiting, techniques include the use of an additional roller to place the active on the film surface, spraying or depositing the active onto the film surface, adding the active by either simple (applied to dry backing film) or dual slot die extrusion (backing film and particles formed simultaneously) and the like. The active may be placed on either or both of the opposed film surfaces, i.e., the top and/or bottom film surfaces by deposition techniques. Deposition techniques would include the ability to accurately meter the amount of active onto the surface of the film. In some embodiments, the active may be dispersed in a fluid medium and the dispersion deposited on the film, such as in a coating layer. Desirably, the active particles are securably disposed onto the film, such as being embedded into the film. Moreover, such particles are desirably not fully encased or fully embedded into the film, but remain exposed to the surface of the film, such as in the case where the particles are partially embedded or partially encased.

Monitoring and control of the thickness of the film also contributes to the production of a uniform film by providing a film of uniform thickness. The thickness of the film may be monitored with gauges such as Gamma or Beta Gauges. A gauge may be coupled to another gauge at the end of the drying apparatus, i.e. drying oven or tunnel, to communicate through feedback loops to control and adjust the opening in the coating apparatus, resulting in control of uniform film thickness. Alternatively, the thickness of the film can also be controlled by manual measurement during the production process to achieve the desired thickness of the film.

The film products are generally formed by combining a properly selected polymer and polar solvent, as well as any agent or filler as desired. Desirably, the solvent content of the combination is at least about 30% by weight of the total combination. The material formed by this combination is formed into a film, desirably by roll coating, and then dried, desirably by a rapid and controlled drying process to maintain the uniformity of the film, more specifically, a non-self-aggregating uniform heterogeneity. The resulting film will desirably contain about ten percent (10%) by weight or less solvent, more desirably about eight percent (8%) by weight or less solvent, even more desirably about six percent (6%) by weight or less solvent and most desirably about two percent (2%) or less solvent. The solvent may be water, a polar organic solvent including, but not limited to, ethanol, isopropanol, acetone, methylene chloride, or any combination thereof.

Consideration of the above discussed parameters, such as, but not limited to, rheology properties, viscosity, mixing method, casting method and drying method, also impact material selection for the different components of the present invention. Furthermore, such consideration with proper material selection provides the compositions of the present invention, including a pharmaceutical, biological, bioeffecting and/or cosmetic dosage form or film product having no more than a ten percent (10%) by weight variance of an active, e.g. a pharmaceutical, biological, bioeffecting and/or cosmetic active per unit weight, or no more of than a ten percent (10%) variance by weight of an active per unit weight of the film product. In other words, the uniformity of the present invention is determined by the presence of no more than a ten percent (10%) by weight of the pharmaceutical, biological, bioeffecting, active component and/or cosmetic variance throughout the matrix. Desirably, the variance is less than five percent (5%) by weight, less than two percent (2%) by weight, less than one percent (1%) by weight, or less than 0.5% by weight.

### Film-Forming Polymers For the Film

The film units or dosages of the present invention include at least one water soluble polymer. The films may also include water swellable or water insoluble polymers, if desired.

In some embodiments, the self-supporting film includes a saccharide-based polymer, which is water soluble. For example, the saccharide-based polymer may be cellulose or a cellulose derivative. Specific examples of useful saccharide-based, water soluble polymers include, but are not limited to, polydextrose, pullulan, hydroxypropylmethyl cellulose (HPMC), hydroxyethyl cellulose (HPC), hydroxypropyl cellulose, carboxymethyl cellulose, sodium aginate, xanthan gum, tragancanth gum, guar gum, acacia gum, arabic gum, starch, gelatin, and combinations thereof.

In some preferred embodiments, the saccharide-based polymer may be at least one cellulosic polymer, polydextrose, or combinations thereof. The film may also include non-saccharide-based, water soluble or water insoluble polymers. Examples of non-saccharide based, water soluble polymers include polyethylene oxide, polyvinylpyrrolidone, polyvinyl alcohol, polyethylene glycol, polyacrylic acid, methylmethacrylate copolymer, carboxyvinyl copolymers, and combinations thereof. Specific examples of useful water insoluble polymers include, but are not limited to, ethyl cellulose, hydroxypropyl ethyl cellulose, cellulose acetate phthalate, hydroxypropyl methyl cellulose phthalate and combinations thereof.

In some further preferred embodiments, the polymer may be a combination of hydroxypropylmethyl cellulose and polyethylene oxide. In some other preferred embodiments, the polymer is a combination of polydextrose and polyethylene oxide. In still further preferred embodiments, the polymer is a combination of polydextrose, hydroxy propylmethyl cellulose and polyethylene oxide.

As used herein, the phrase "water soluble polymer" and variants thereof refer to a polymer that is at least partially soluble in water, and desirably fully or predominantly soluble in water, or absorbs water. In some embodiments, the film unit of the present invention is at least partially dissolvable when exposed to a wetting agent. In some other embodiments, the inventive film unit is substantially dissolvable when exposed to a wetting agent.

Polymers that absorb water are often referred to as being water swellable polymers. The materials useful with the present invention may be water soluble or water swellable at room temperature and other temperatures, such as temperatures exceeding room temperature. Moreover, the materials may be water soluble or water swellable at pressures less than atmospheric pressure. Desirably, the water soluble polymers are water soluble or water swellable having at least 20 percent by weight water uptake. Water swellable polymers having a twenty-five (25) or greater percent by weight water uptake are also useful. Films or dosage forms of the present invention formed from such water soluble polymers are desirably sufficiently water soluble to be dissolvable upon contact with bodily fluids.

Other polymers useful for incorporation into the films of the present invention include biodegradable polymers, copolymers, block polymers and combinations thereof. Among the known useful polymers or polymer classes which meet the above criteria are: poly(glycolic acid) (PGA), poly(lactic acid) (PLA), polydioxanoes, polyoxalates, poly(α-esters), polyanhydrides, polyacetates, polycaprolactones, poly(orthoesters), polyamino acids, polyaminocarbonates, polyurethanes, polycarbonates, polyamides, poly(alkyl cyanoacrylates), and mixtures and copolymers thereof. Additional useful polymers include, stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkanoates and mixtures thereof. Binary and ternary systems are contemplated.

Other specific polymers useful include those marketed under the Medisorb and Biodel trademarks. The Medisorb materials are marketed by the Dupont Company of Wilmington, Delaware and are generically identified as a "lactide/glycolide co-polymer" containing "propanoic acid, 2-hydroxy-polymer with hydroxy-polymer with hydroxyacetic acid." Four such polymers include lactide/glycolide 100L, believed to be 100% lactide having a melting point within the range of 338°-347°F (170°-175°C); lactide/glycolide 100L, believed to be 100% glycolide having a melting point within the range of 437°-455°F (225°-235°C); lactide/glycolide 85/15, believed to be 85% lactide and 15% glycolide with a melting point within the range of 338°-347°F (170°-175° C); and lactide/glycolide 50/50, believed to be a copolymer of 50% lactide and 50% glycolide with a melting point within the range of 338°-347°F (170°-175°C).

The Biodel materials represent a family of various polyanhydrides which differ chemically.

Although a variety of different polymers may be used, it is desired to select polymers to provide a desired viscosity of the mixture prior to drying. For example, if the agent or other components are not soluble in the selected solvent, a polymer that will provide a greater viscosity is desired to assist in maintaining uniformity. On the other hand, if the components are soluble in the solvent, a polymer that provides a lower viscosity may be preferred.

The polymer plays an important role in affecting the viscosity of the film. Viscosity is one property of a liquid that controls the stability of the agent in an emulsion, a colloid or a suspension. Generally the viscosity of the matrix will vary from about 400 cps to about 100,000 cps, preferably from about 800 cps to about 60,000 cps, and most preferably from about 1,000 cps to about 40,000 cps. Desirably, the viscosity of the film-forming matrix will rapidly increase upon initiation of the drying process.

The viscosity may be adjusted based on the selected agent component and/or active, depending on the other components within the matrix. For example, if the component is not soluble within the selected solvent, a proper viscosity may be selected to prevent the component from settling which would adversely affect the uniformity of the resulting film. The viscosity may be adjusted in different ways. To increase viscosity of the film matrix, the polymer may be chosen of a higher molecular weight or crosslinkers may be added, such as salts of calcium, sodium and potassium. The viscosity may also be adjusted by adjusting the temperature or by adding a viscosity increasing component. Components that will increase the viscosity or stabilize the emulsion/suspension include higher molecular weight polymers and polysaccharides and gums, which include without limitation, alginate, carrageenan, hydroxypropyl methyl cellulose, locust bean gum, guar gum, xanthan gum, dextran, gum arabic, gellan gum and combinations thereof.

It has also been observed that certain polymers which when used alone would ordinarily require a plasticizer to achieve a flexible film, can be combined without a plasticizer and yet achieve flexible films. For example, HPMC and HPC when used in combination provide a flexible, strong film with the appropriate plasticity and elasticity for manufacturing and storage. No additional plasticizer or polyalcohol is needed for flexibility.

Additionally, polyethylene oxide (PEO), when used alone or in combination with a hydrophilic cellulosic polymer and/or polydextrose, achieves flexible, strong films. Additional plasticizers or polyalcohols are not needed for flexibility. Non-limiting examples of suitable cellulosic polymers for combination with PEO include HPC and HPMC. PEO and HPC have essentially no gelation temperature, while HPMC has a gelation temperature of 58-64°C (Methocel EF available from Dow Chemical Co.). Moreover, these films are sufficiently flexible even when substantially free of organic solvents, which may be removed without compromising film properties. Organic solvents may tend to plasticize the film, so leaving out organic solvents may be useful when this effect is less desirable or is to be controlled by other additives. PEO based films also exhibit good resistance to tearing, little or no curling, and fast dissolution rates when the polymer component contains appropriate levels of PEO.

To achieve the desired film properties, the level and/or molecular weight of PEO in the polymer component may be varied. Modifying the PEO content affects properties such as tear resistance, dissolution rate, and adhesion tendencies. Thus, one method for controlling film properties is to modify the PEO content. For instance, in some embodiments rapid dissolving films are desirable. By modifying the content of the polymer component, the desired dissolution characteristics can be achieved.

In accordance with the present invention, PEO desirably ranges from about 20% to 100% by weight in the polymer component. In some embodiments, the amount of PEO desirably ranges from about 1mg to about 200mg. The hydrophilic cellulosic polymer and/or polydextrose ranges from about 0% to about 80% by weight, or in a ratio of up to about 4:1 with the PEO, and desirably in a ratio of about 1:1.

In some embodiments, it may be desirable to vary the PEO levels to promote certain film properties. To obtain films with high tear resistance and fast dissolution rates, levels of about 50% or greater of PEO in the polymer component are desirable. To achieve adhesion prevention, i.e., preventing the film from adhering to the roof of the mouth, PEO levels of about 20% to 75% are desirable. In some embodiments, however, adhesion to the roof of the mouth may be desired, such as for administration to animals or children. In such cases, higher levels of PEO may be employed. More specifically, structural integrity and dissolution of the film can be controlled such that the film can adhere to mucosa and be readily removed, or adhere more firmly and be difficult to remove, depending on the intended use.

The molecular weight of the PEO may also be varied. High molecular weight PEO, such as about 4 million, may be desired to increase mucoadhesion of the film. More desirably, the molecular weight may range from about 100,000 to 900,000, more desirably from about 100,000 to 600,000, and most desirably from about 100,000 to 300,000. In some embodiments, it may be desirable to combine high molecular weight (600,000 to 900,000) with low molecular weight (100,000 to 300,000) PEOs in the polymer component.

For instance, certain film properties, such as fast dissolution rates and high tear resistance, may be attained by combining small amounts of high molecular weight PEOs with larger amounts of lower molecular weight PEOs. Desirably, such compositions contain about 60% or greater levels of the lower molecular weight PEO in the PEO-blend polymer component.

To balance the properties of adhesion prevention, fast dissolution rate, and good tear resistance, desirable film compositions may include about 50% to 75% low molecular weight PEO, optionally combined with a small amount of a higher molecular weight PEO, with the remainder of the polymer component containing a hydrophilic cellulosic polymer (HPC or HPMC) and/or polydextrose.

In some embodiments the film may include polyvinyl alcohol (PVA), alone or in combination with at least one additional polymer Examples of an additional polymer include a cellulosic polymer, starch, polyvinyl pyrrolidone (PVP), polyethylene oxide (PEO), an alginate, a pectin, or combinations thereof. PVA can be used in the films to improve film strength and/or to vary and slow dissolution times. The films are especially useful for the delivery of cosmetics, nutraceuticals, biologics, pharmaceuticals and bioeffecting agents. In a preferred embodiment, the film includes PVA without any added plasticizers. For example, the film can include both PVA, which provides strength to the film and PEO, which provides flexibility to the film and nay obviate the need for a plasticizer.

PVA can be used in varying amounts depending upon the product application and characteristics desired. For example, in general, a larger amount of PVA will increase film strength and increase dissolution time. For films that require high active dosing, PVA can be used effectively at minimum amount of 0.5, preferably 1%, more preferably 5%, by weight of the film, to improve film strength. The PVA an be effectively used at a maximum amount, for example, 80%, preferably 50%, more preferably 25% by weight of the film. For slowing dissolution time, PVA can be used at levels as high as 80%. A film containing an active can be coated on one or both surfaces with a PVA containing layer to modify the dissolution of the film and the release of an active from the film.

High loading of actives can decrease the strength and flexibility of the film. Including PVA in the film either alone or in combination with at least one other polymer can increase the tensile strength of the film. Also, drug particles or taste-masked or coated or modified release drug particles may have a larger particle size, which can make loading of these particles into the film difficult. PVA can increase the viscosity of the film solution to allow improved drug loading.

When an active is introduced to the film, the amount of active per unit area is determined by the uniform distribution of the film. In accordance with the present invention, the ingestible film has a predetermined dosage of the active component.

For example, when the films are cut into individual dosage forms, the amount of the active in the dosage form can be known with a great deal of accuracy. This is achieved because the amount of the active in a given area is substantially identical to the amount of active in an area of the same dimensions in another part of the film. The accuracy in dosage is particularly advantageous when the active is a medicament, i.e. a drug.

### Film-Forming Polymers For the Film

The active components that may be incorporated into the films of the present invention include, without limitation pharmaceutical and cosmetic actives, drugs, medicaments, proteins, antigens or allergens such as ragweed pollen, spores, microorganisms, seeds, mouthwash components, flavors, fragrances, enzymes, preservatives, sweetening agents, colorants, spices, vitamins and combinations thereof.

A wide variety of medicaments, bioactive active substances and pharmaceutical compositions may be included in the dosage forms of the present invention. Examples of useful drugs include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of medicating active ingredients contemplated for use in the present invention include antacids, H₂-antagonists, and analgesics. For example, antacid dosages can be prepared using the ingredients calcium carbonate alone or in combination with magnesium hydroxide, and/or aluminum hydroxide. Moreover, antacids can be used in combination with H₂-antagonists.

Analgesics include opiates and opiate derivatives, such as oxycodone (commercially available as Oxycontin®); ibuprofen (commercially available as Motrin®, Advil®, Motrin Children's®, Motrin IB®, Advil Children's®, Motrin Infants'®, Motrin Junior®, Ibu-2®, Proprinal®, Ibu-200®, Midol Cramp Formula®, Bufen®, Motrin Migraine Pain®, Addaprin® and Haltran®), aspirin (commercially available as Empirin®, Ecotrin®, Genuine Bayer®, and Halfprin®), acetaminophen (commercially available as Silapap Infant's®, Silapap Children's®, Tylenol®, Tylenol Children's®, Tylenol Extra Strength®, Tylenol Infants' Original®, Tylenol Infants'®, Tylenol Arthritis®, T-Painol®, Q-Pap®, Cetafen®, Dolono®, Tycolene®, APAP® and Aminofen®), and combinations thereof that may optionally include caffeine. Other pain relieving agents may be used in the present invention, including meperidine hydrochloride (commercially available as Demerol®), capsaicin (commercially available as Qutenza®), morphine sulfate and naltrexone hydrochloride (commercially available as Embeda®), hydromorphone hydrochloride (commercially available as Dilaudid®), propoxyphene napsylate and acetaminophen (commercially available as Darvocet-N®), Fentanyl (commercially available as Duragesic®, Onsolis®, and Fentora®), sodium hyaluronate (commercially avialble as Euflexxa®), adalimumab (commercially available as Humira®), sumatriptan succinate (commercially available as Imitrex®), fentanyl iontophoretic (commercially available as Ionsys®), orphenadrine citrate (commercially available as Norgesic®), magnesium salicylate tetrahydrate (commercially available as Novasal®), oxymorphone hydrochloride (commercially available as Opana ER®), methocarbamol (commercially available as Robaxin®), carisoprodol (commercially available as Soma®), tramadol hydrochloride (commercially available as Ultracet® and Ultram®), morphine sulfate (commercially available as MS Contin®), metaxalone (commercially available as Skelaxin®), oxycodone hydrochloride (commercially available as OxyContin®), acetaminophen/oxycodone hydrochloride (commercially available as Percocet®), oxycodone/aspirin (commercially available as Percodan®), hydrocodone bitartrate/acetaminophen (commercially available as Vicodin®), hydrocodone bitartrate/ibuprofen (commercially available as Vicoprofen®), nepafenac (commercially available as Nevanac®), and pregabalin (commercially available as Lyrica®).

The present invention may further include agents such as NSAIDs, including etodolac (commercially available as Lodine®), ketorolac tromethamine (commercially available as Acular® or Acuvail®), naproxen sodium (commercially available as Anaprox®, Naprosyn®), flurbiprofen (commercially available as Ansaid®), diclofenac sodium/misoprostol (commercially available as Arthrotec®), celecoxib (commercially available as Celebrex®), sulindac (commercially available as Clinoril®), oxaprozin (commercially available as Daypro®), piroxicam (commercially available as Feldene®), indomethacin (commercially available as Indocin®), meloxicam (commercially available as Mobic®), mefenamic acid (commercially available as Ponstel®), tolmetin sodium (commercially available as Tolectin®), choline magnesium trisalicylate (commercially available as Trilisate®), diclofenac sodium (commercially available as Voltaren®), diclofenac potassium (commercially available as Cambia® or Zipsor®), and misoprostol (commercially available as Cytotec®). Opiate agonists and antagonists, such as buprenorphine and naloxone are further examples of drugs for use in the present invention.

Other preferred drugs for other preferred active ingredients for use in the present invention include anti-diarrheals such as loperamide (commercially available as Imodium AD®, Imotil®, Kaodene®, Imperim®, Diamode®, QC Anti-Diarrheal®, Health Care America Anti-Diarrheal®, Leader A-D®, and Imogen®), nitazoxanide (commercially available as Alinia®) and diphenoxylate hydrochloride/atropine sulfate (commercially available as Lomotil®), anti-histamines, anti-tussives, decongestants, vitamins, and breath fresheners. Common drugs used alone or in combination for colds, pain, fever, cough, congestion, runny nose and allergies, such as acetaminophen, ibuprofen, chlorpheniramine maleate, dextromethorphan, dextromethorphan HBr, phenylephrine HCl, pseudoephedrine HCl, diphenhydramine and combinations thereof, such as dextromethophan HBr and phenylephrine HCl (available as Triaminic®) may be included in the film compositions of the present invention.

Other active agents useful in the present invention include, but are not limited to alcohol dependence treatment, such as acamprosate calcium (commercially available as Campral®); Allergy treatment medications, such as promethazine hydrochloride (commercially available as Phenergan®), bepotastine besilate (commercially available as Bepreve®), hydrocodone polistirex/chlorpheniramine polistirex (commercially available as Tussionex®), cetirizine hydrochloride (commercially available as Zyrtec®), cetirizine hydrochloride/pseudoephedrine hydrochloride (commercially available as Zyrtec-D®), promethazine hydrochloride/codeine phosphate (commercially available as Phenergan® with Codeine), pemirolast (commercially available as Alamast®), fexofenadine hydrochloride (commercially available as Allegra®), meclizine hydrochloride (commercially available as Antivert®), azelastine hydrochloride (commercially available as Astelin®), nizatidine (commercially available as Axid®), desloratadine (commercially available as Clarinex®), cromolyn sodium (commercially available as Crolom®), epinastine hydrochloride (commercially available as Elestat®), azelastine hydrochloride (commercially available as Optivar®), prednisolone sodium phosphate (commercially available as Orapred ODT®), olopatadine hydrochloride (commercially available as Patanol®), ketotifen fumarate (commercially available as Zaditor®), and montelukast sodium (commercially available as Singulair®); and anti-histamines such as diphenhydramine HCl (available as Benadryl®), loratadine (available as Claritin®), astemizole (available as Hismanal®), nabumetone (available as Relafen®), diphenydramine HCL (available as TheraFlu®) and clemastine (available as Tavist®).

Films of the present invention may further include Alzheimer's treatment medications, such as tacrine hydrochloride (commercially available as Cognex®), galantamine (commercially available as Razadyne®), donepezil hydrochloride (commercially available as Aricept®), rivastigmine tartrate (commercially available as Exelon®), caprylidene (commercially available as Axona®), and memantine (commercially available as Namenda®); anemia medication, such as cyanocobalamin (commercially available as Nascobal®) and ferumoxytol (commercially available as Feraheme®); anesthetics, such as antipyrine with benzocaine (commercially available as Auralgan®, Aurodex® and Auroto®); angina medication, such as amlodipine besylate (commercially available as Norvasc®), nitroglycerin (commercially available as Nitro-Bid®, Nitro-Dur®, Nitrolingual®, Nitrostat®, Transderm-Nitro®), isosorbide mononitrate (commercially available as Imdur®), and isosorbide dinitrate (commercially available as Isordil®); anti-tussives such as guaifensin; anti-Alzheimer's agents, such as nicergoline; and Ca^{H}-antagonists such as nifedipine (commercially available as Procardia® and Adalat®).

Actives useful in the present invention may also include anti-asthmatics, such as albuterol sulfate (commercially available as Proventil®), ipratropium bromide (commercially available as Atrovent®), salmeterol xinafoate (commercially available as Serevent®), zafirlukast (commercially available as Accolate®), flunisolide (commercially available as AeroBid®), metaproterenol sulfate (commercially available as Alupent®), albuterol inhalation (commercially available as Ventolin®), terbutaline sulfate (commercially available as Brethine®), formoterol (commercially available as Foradil®), cromolyn sodium (commercially available as Intal®), levalbuterol hydrochloride (commercially available as Xopenex®), zileuton (commercially available as Zyflo®), fluticasone propionate/salmeterol (commercially available as Advair®), albuterol sulfate/triamcinolone acetonide (commercially available as Azmacort®), dimethylxanthine (commercially available as Theophylline®), and beclomethasone (commercially available as Beclovent®, Beconase®, Qvar®, Vancenase®, Vanceril®); angioedema medication, such as C1 esterase Inhibitor (human) (commercially available as Berinert®) and ecallantide (commercially available as Kalbitor®); and antibacterial medications, such as trimethoprim/sulfamethoxazole (commercially available as Bactrim®), mupirocin (commercially available as Bactroban®), metronidazole (commercially available as Flagyl®), sulfisoxazole acetyl (commercially available as Gantrisin®), bismuth subsalicylate and metronidazole/tetracycline hydrochloride (commercially available as Helidac Therapy®), nitrofurantoin (commercially available as Macrodantin®), norfloxacin (commercially available as Noroxin®), erythromycin ethylsuccinate/Sulfisoxazole acetyl (commercially available as Pediazole®), and levofloxacin (commercially available as Levaquin®).

The present invention may further include one or more Antibiotics, including amoxicillin (commercially available as Amoxil®), ampicillin (commercially available as Omnipen®, Polycillin® and Principen®), amoxicillin/clavulanate potassium (commercially available as Augmentin®), moxifloxacin hydrochloride (commercially available as Avelox®), besifloxacin (commercially available as Besivance®), clarithromycin (commercially available as Biaxin®), ceftibuten (commercially available as Cedax®), cefuroxime axetil (commercially available as Ceftin®), cefprozil (commercially available as Cefzil®), ciprofloxacin hydrochloride (commercially available as Ciloxan® and Cipro®), clindamycin phosphate (commercially available as Cleocin T®), doxycycline hyclate (commercially available as Doryx®), dirithromycin (commercially available as Dynabac®), erythromycin (commercially available as E.E.S. ®, E-Mycin®, Eryc®, Ery-Tab®, Erythrocin®, and PCE®), erythromycin topical (commercially available as A/T/S®, Erycette®, T-Stat®), gemifloxacin (commercially available as Factive®), ofloxacin (commercially known as Ocuflox®, Floxin®), telithromycin (commercially available as Ketek®), lomefloxacin hydrochloride (commercially available as Maxaquin®), minocycline hydrochloride (commercially available as Minocin®), fosfomycin tromethamine (commercially available as Monurol®), penicillin with potassium (commercially available as Penicillin VK®, Veetids®), trimethoprim (commercially available as Primsol®), ciprofloxacin hydrochloride (commercially available as Proquin XR®), rifampin, isoniazid and pyrazinamide (commercially available as Rifater®), cefditoren (commercially available as Spectracef®), cefixime (commercially available as Suprax®), tetracycline (commercially available as Achromycin V® and Sumycin®), tobramycin (commercially available as Tobrex®), rifaximin (commercially available as Xifaxan®), azithromycin (commercially available as Zithromax®), azithromycin suspension (commercially available as Zmax®), linezolid (commercially available as Zyvox®), benzoyl peroxide and clindamycin (commercially available as BenzaClin®), erythromycin and benzoyl peroxide (commercially available as Benzamycin®), dexamethasone (commercially available as Ozurdex®), ciprofloxacin and dexamethasone (commercially available as Ciprodex®), polymyxin B sulfate/neomycin sulfate/hydrocortisone (commercially available as Cortisporin®), colistin sulfate/neomycin sulfate/hydrocortisone acetate/thonzonium bromide (commercially available as Cortisporin-TC Otic®), cephalexin hydrochloride (commercially available as Keflex®), cefdinir (commercially available as Omnicef®), and gatifloxacin (commercially available as Zymar®).

Other useful actives include cancer treatment medications, including cyclophosphamide (commercially available as Cytoxan®), methotrexate (commercially available as Rheumatrex® and Trexal®), tamoxifen citrate (commercially available as Nolvadex®), bevacizumab (commercially available as Avastin®), everolimus (commercially available as Afinitor®), pazopanib (commercially available as Votrient®), and anastrozole (commercially available as Arimidex®); leukemia treatment, such as ofatumumab (commercially available as Arzerra®); anti-thrombotic drugs, such as antithrombin recombinant lyophilized powder (commercially available as Atryn®), prasugrel (commercially available as Efient®); anti-coagulants, such as aspirin with extended-release dipyridamole (commercially available as Aggrenox®), warfarin sodium (commercially available as Coumadin®), dipyridamole (commercially available as Persantine®), dalteparin (commercially available as Fragmin®), danaparoid (commercially available as Orgaran®), enoxaparin (commercially available as Lovenox®), heparin (commercially available as Hep-Lock, Hep-Pak, Hep-Pak CVC, Heparin Lock Flush), tinzaparin (commercially available as Innohep®), and clopidogrel bisulfate (commercially available as Plavix®); antiemetics, such as granisetron hydrochloride (commercially available as Kytril®) and nabilone (commercially available as Cesamet®), trimethobenzamide hydrochloride (commercially available as Tigan®), and ondansetron hydrochloride (commercially available as Zofran®); anti-fungal treatment, such as ketoconazole (commercially available as Nizoral®), posaconazole (commercially available as Noxafil®), ciclopirox (commercially available as Penlac®), griseofulvin (commercially available as Gris-PEG®), oxiconazole nitrate (commercially available as Oxistat®), fluconazole (commercially available as Diflucan®), sertaconazole nitrate (commercially available as Ertaczo®), terbinafine hydrochloride (commercially available as Lamisil®), ciclopirox (commercially available as Loprox®), nystatin/triamcinolone acetonide (commercially available as Mycolog-II®), econazole nitrate (commercially available as Spectazole®), itraconazole (commercially available as Sporanox®), and terconazole (commercially available as Terazol®).

Active agents may further include anti-inflammatory medications, such as hydroxychloroquine sulfate (commercially available as Plaquenil®), fluticasone propionate (commercially available as Cutivate®), canakinumab (commercially available as Llaris®), amcinonide (commercially available as Cyclocort®), methylprednisolone (commercially available as Medrol®), budesonide (commercially available as Entocort EC®), anakinra (commercially available as Kineret®), diflorasone diacetate (commercially available as Psorcon®), and etanercept (commercially available as Enbrel®); antispasmodic medication, such as phenobarbital/hyoscyamine sulfate/atropine sulfate/scopolamine hydrobromide (commercially available as Donnatal®); antiviral treatment, such as oseltamivir phosphate (commercially available as Tamiflu®); anti-parasites medication, including tinidazole (commercially available as Tindamax®); appetite treatment mediations, such as megestrol acetate (commercially available as Megace ES®), phentermine hydrochloride (commercially available as Adipex-P®), and diethylpropion hydrochloride (commercially available as Tenuate®); arthritis medications, including leflunomide (commercially available as Arava®), certolizumab pegol (commercially available as Cimzia®), diclofenac sodium (commercially available as Pennsaid®), golimumab (commercially available as Simponi®), and tocilizumab (commercially available as Actemra®); bladder control medication, such as trospium chloride (commercially available as Sanctura®), desmopressin acetate (commercially available as DDAVP®), tolterodine tartrate (commercially available as Detrol®), oxybutynin chloride (commercially available as Ditropan® or Gelnique®), darifenacin (commercially available as Enablex®), and solifenacin succinate (commercially available as VESIcare®); blood vessel constrictors, such as methylergonovine maleate (commercially available as Methergine®); plasma uric managers, such as rasburicase (commercially available as Elitek®); iron deficiency anemia medications, such as ferumoxytol (commercially available as Feraheme®); lymphoma medications, such as pralatrexate (commercially available as Folotyn®), romidepsin (commercially available as Isodax®); malaria medication, such as artemether/lumefantrine (commercially available as Coartem®); hyponatremia medication, such as tolvatpan (commercially available as Samsca®); medication for treatment of von Willebrand disease (commercially available as Wilate®); anti-hypertension medications, such as treprostinil (commercially available as Tyvaso®), tadalafil (commercially available as Adcirca®); cholesterol lowering medication, including paricalcitol (commercially available as Altocor®), pitavastatin (commercially available as Livalo®), lovastatin, niacin (commercially available as Advicor®), colestipol hydrochloride (commercially available as Colestid®), rosuvastatin calcium (commercially available as Crestor®), fluvastatin sodium (commercially available as Lescol®), atorvastatin calcium (commercially available as Lipitor®), lovastatin (commercially available as Mevacor®), niacin (commercially available as Niaspan®), pravastatin sodium (commercially available as Pravachol®), pavastatin sodium with buffered aspirin (commercially available as Pravigard PAC®), cholestyramine (commercially available as Questran®), simvastatin and niacin (commercially available as Simcor®), atenolol, chlorthalidone (commercially available as Tenoretic®), atenolol (commercially available as Tenormin®), fenofibrate (commercially available as Tricor®), fenofibrate (commercially available as Triglide®), ezetimibe/simvastatin (commercially available as Vytorin®), colesevelam (commercially available as WelChol®), bisoprolol fumarate (commercially available as Zebeta®), ezetimibe (commercially available as Zetia®), bisoprolol fumarate/hydrochlorothiazide (commercially available as Ziac®), and simvastatin (commercially available as Zocor®).

The actives included herein may also include chronic kidney disease medication, such as paricalcitol (commercially available as Zemplar®); contraceptive agents, including etonogestrel (commercially available as Implanon®), norethindrone acetate, ethinyl estradiol (commercially available as Loestrin 24 FE®), ethinyl estradiol, norelgestromin (commercially available as Ortho Evra®), levonorgestrel (commercially available as Plan B®), levonorgestrel and ethinyl estradiol (commercially available as Preven®), levonorgestrel, ethinyl estradiol (commercially available as Seasonique®), and medroxyprogesterone acetate (commercially available as Depo-Provera®); COPD medication, such as arformoterol tartrate (commercially available as Brovana®) and ipratropium bromide, albuterol sulfate (commercially available as Combivent®); cough suppressants, including benzonatate (commercially available as Tessalon®), guaifenesin, codeine phosphate (commercially available as Tussi-Organidin NR®), and acetaminophen, codeine phosphate (commercially available as Tylenol with Codeine®); medication for the treatment of diabetes, including pioglitazone hydrochloride, metformin hydrochloride (commercially available as ACTOplus met®), bromocriptine mesylate (commercially available as Cycloset®), liraglutide (commercially available as Victoza®), saxagliptin (commercially available as Onglyza®), pioglitazone hydrochloride (commercially available as Actos®), glimepiride (commercially available as Amaryl®), rosiglitazone maleate, metformin hydrochloride (commercially available as Avandamet®), rosiglitazone maleate (commercially available as Avandaryl®), rosiglitazone maleate (commercially available as Avandia®), exenatide (commercially available as Byetta®), chlorpropamide (commercially available as Diabinese®), pioglitazone hydrochloride, glimepiride (commercially available as Duetact®), metformin hydrochloride (commercially available as Glucophage®), glipizide (commercially available as Glucotrol®), glyburide, metformin (commercially available as Glucovance®), metformin hydrochloride (commercially available as Glumetza®), sitagliptin (commercially available as Januvia®), detemir (commercially available as Levemir®), glipizide, metformin hydrochloride (commercially available as Metaglip®), glyburide (commercially available as Micronase®), repaglinide (commercially available as Prandin®), acarbose (commercially available as Precose®), nateglinide (commercially available as Starlix®), pramlintide acetate (commercially available as Symlin®), and tolazamide (commercially available as Tolinase®).

Other useful agents of the present invention may include digestive agents, such as sulfasalazine (commercially available as Azulfidine®), rabeprazole sodium (commercially available as AcipHex®), lubiprostone (commercially available as Amitiza®), dicyclomine hydrochloride (commercially available as Bentyl®), sucralfate (commercially available as Carafate®), lactulose (commercially available as Chronulac®), docusate (commercially available as Colace®), balsalazide disodium (commercially available as Colazal®), losartan potassium (commercially available as Cozaar®), olsalazine sodium (commercially available as Dipentum®), chlordiazepoxide hydrochloride, clidinium bromide (commercially available as Librax®), esomeprazole magnesium (commercially available as Nexium®), famotidine (commercially available as Pepcid®), lansoprazole (commercially available as Prevacid®), lansoprazole and naproxen (commercially available as Prevacid NapraPAC®), amoxicillin/clarithromycin/lansoprazole (commercially available as Prevpac®), omeprazole (commercially available as Prilosec®), pantoprazole sodium (commercially available as Protonix®), metoclopramide hydrochloride (commercially available as Reglan® or Metozolv®), cimetidine (commercially available as Tagamet®), ranitidine hydrochloride (commercially available as Zantac®), and omeprazole, sodium bicarbonate (commercially available as Zegerid®); diuretics, including spironolactone, hydrochlorothiazide (commercially available as Aldactazide®), spironolactone (commercially available as Aldactone®). bumetanide (commercially available as Bumex®), torsemide (commercially available as Demadex®), chlorothiazide (commercially available as Diuril®), furosemide (commercially available as Lasix®), metolazone (commercially available as Zaroxolyn®), and hydrochlorothiazide, triamterene (commercially available as Dyazide®).

Agents useful herein may also include treatment for emphysema, such as tiotropium bromide (commercially available as Spiriva®); fibromyalgia medication, such as milnacipran hydrochloride (commercially available as Savella®); medication for the treatment of gout, such as colchicine (commercially available as Colcrys®), and febuxostat (commercially available as Uloric®); enema treatments, including aminosalicylic acid (commercially available as Mesalamine® and Rowasa®); epilepsy medications, including valproic acid (commercially available as Depakene®), felbamate (commercially available as Felbatol®), lamotrigine (commercially available as Lamictal®), primidone (commercially available as Mysoline®), oxcarbazepine (commercially available as Trileptal®), zonisamide(commercially available as Zonegran®), levetiracetam (commercially available as Keppra®), and phenytoin sodium (commercially available as Dilantin®).

Erectile dysfunction therapies useful herein include, but are not limited to, drugs for facilitating blood flow to the penis, and for effecting autonomic nervous activities, such as increasing parasympathetic (cholinergic) and decreasing sympathetic (adrenersic) activities. Useful agents for treatment of erectile dysfunction include, for example, those agents available as alprostadil (commercially available as Caverject®), tadalafil (commercially available as Cialis®), vardenafil (commercially available as Levitra®), apomorphine (commercially available as Uprima®), yohimbine hydrochloride (commercially available as Aphrodyne®, Yocon®), and sildenafil citrate (commercially available as Viagra®).

Agents useful herein may further include eye medications and treatment, such as dipivefrin hydrochloride (commercially available as Propine®), valganciclovir (commercially available as Valcyte®), ganciclovir ophthalmic gel (commercially available as Zirgan®); bepotastine besilate (commercially available as Bepreve®), besifloxacin (commercially available as Besivance®), bromfenac (commercially available as Xibrom®), fluorometholone (commercially available as FML®), pilocarpine hydrochloride (commercially available as Pilocar®), cyclosporine (commercially available as Restasis®), brimonidine tartrate (commercially available as Alphagan P®), dorzolamide hydrochloride/timolol maleate (commercially available as Cosopt®), bimatoprost (commercially available as Lumigan®), timolol maleate (available as Timoptic®), travoprost (commercially available as Travatan®), latanoprost (commercially available as Xalatan®), echothiophate iodide (commercially available as Phospholine Iodide®), and ranibizumab (commercially available as Lucentis®); fluid controllers, such as acetazolamide (commercially available as Diamox®); gallstone medications, including ursodiol (commercially available as Actigall®); medication for the treatment of gingivitis, including chlorhexidine gluconate (commercially available as Peridex®); headache medications, including butalbital/codeine phosphate/aspirin/caffeine (commercially available as Fiornal® with Codeine), naratriptan hydrochloride (commercially available as Amerge®), almotriptan (commercially available as Axert®), ergotamine tartrate/caffeine (commercially available as Cafergot®), butalbital/acetaminophen/caffeine (commercially available as Fioricet®), butalbital/aspirin/caffeine (commercially available as Fiorinal®), frovatriptan succinate (commercially available as Frova®), rizatriptan benzoate (commercially available as Maxalt®), isometheptene mucate/dichloralphenazone/acetaminophen (commercially available as Midrin®), dihydroergotamine mesylate (commercially available as Migranal®), eletriptan hydrobromide (commercially available as Relpax®), and zolmitriptan (commercially available as Zomig®); influenza medication, such as haemophilus b conjugate vaccine; tetanus toxoid conjugate (commercially available as Hiberix®); and heart treatments, including quinidine sulfate, isosorbide dinitrate/hydralazine hydrochloride (commercially available as BiDil®), digoxin (commercially available as Lanoxin®), flecainide acetate (commercially available as Tambocor®), mexiletine hydrochloride (commercially available as Mexitil®), disopyramide phosphate (commercially available as Norpace®), procainamide hydrochloride (commercially available as Procanbid®), and propafenone (commercially available as Rythmol®).

Other useful agents include hepatitis treatments, including entecavir (commercially available as Baraclude®), hepatitis B immune globulin (commercially available as HepaGam B®), and copegus/rebetol/ribasphere/vilona/virazole (commercially available as Ribavirin®); herpes treatments, including valacyclovir hydrochloride (commercially available as Valtrex®), penciclovir (commercially available as Denavir®), acyclovir (commercially available as Zovirax®), and famciclovir (commercially available as Famvir®); treatment for high blood pressure, including enalaprilat (available as Vasotec®), captopril (available as Capoten®) and lisinopril (available as Zestril®), verapamil hydrochloride (available as Calan®), ramipril (commercially available as Altace®), olmesartan medoxomil (commercially available as Benicar®), amlodipine/atorvastatin (commercially available as Caduet®), nicardipine hydrochloride (commercially available as Cardene®), diltiazem hydrochloride (commercially available as Cardizem®), quinapril hydrochloride (commercially available as Accupril®), quinapril hydrochloride/hydrochlorothiazide (commercially available as Accuretic®), perindopril erbumine (commercially available as Aceon®), candesartan cilexetil (commercially available as Atacand®), candesartan cilexetil/hydrochlorothiazide (commercially available as Atacand HCT®), irbesartan/hydrochlorothiazide (commercially available as Avalide®), irbesartan (commercially available as Avapro®), amlodipine besylate/olmesartan medoxomil (commercially available as Azor®), levobunolol hydrochloride (commercially available as Betagan®), betaxolol hydrochloride (commercially available as Betoptic®), nebivolol (commercially available as Bystolic®), captopril/hydrochlorothiazide (commercially available as Capozide®), doxazosin mesylate (commercially available as Cardura®), clonidine hydrochloride (commercially available as Catapres®), carvedilol (commercially available as Coreg®), nadolol (commercially available as Corgard®), nadolol/bendroflumethiazide (commercially available as Corzide®), valsartan (commercially available as Diovan®), isradipine (commercially available as DynaCirc®), Guanabenz acetate. (commercially available as Wytensin ®), Guanfacine hydrochloride (commercially available as Tenex ® or Intuniv®), losartan potassium/hydrochlorothiazide (commercially available as Hyzaar®), propranolol hydrochloride (commercially available as Indera®), propranolol hydrochloride/hydrochlorothiazide (commercially available as Inderide®), eplerenone (commercially available as Inspra®), ambrisentan (commercially available as Letairis®), enalapril maleate/felodipine (commercially available as Lexxel®), metoprolol tartrate (commercially available as Lopressor®), benazepril hydrochloride (commercially available as Lotensin®), benazepril hydrochloride/hydrochlorothiazide (commercially available as Lotensin HCT®), amlodipine/benazepril hydrochloride (commercially available as Lotrel®), indapamide (commercially available as Lozol®), trandolapril (commercially available as Mavik®), telmisartan (commercially available as Micardis®), telmisartan/hydrochlorothiazide (commercially available as Micardis HCT®), prazosin hydrochloride (commercially available as Minipress®), amiloride, hydrochlorothiazide (commercially available as Moduretic®), fosinopril sodium (commercially available as ZZXT Monopril®), fosinopril sodium/hydrochlorothiazide (commercially available as Monopril-HCT®), pindolol (commercially available as Visken®), felodipine (commercially available as Plendil®), sildenafil citrate (commercially available as Revatio®), Nisoldipine (commercially available as Sular®), trandolapril/verapamil hydrochloride (commercially available as Tarka®), aliskiren (commercially available as Tekturna®), eprosartan mesylate (commercially available as Teveten®), eprosartan mesylate/hydrochlorothiazide (commercially available as Teveten HCT®), moexipril hydrochloride/hydrochlorothiazide (commercially available as Uniretic®), moexipril hydrochloride (commercially available as Univasc®), enalapril maleate/hydrochlorothiazide (commercially available as Vaseretic®), and lisinopril/hydrochlorothiazide (commercially available as Zestoretic®).

The present invention may include agents useful in the medication for the treatment of HIV/AIDS, such as amprenavir (commercially available as Agenerase®), tipranavir (commercially available as Aptivus®), efavirenz/emtricitabine/tenofovir (commercially available as Atripla®), lamivudine/zidovudine (commercially available as Combivir®), indinavir sulfate (commercially available as Crixivan®), lamivudine (commercially available as Epivir®), saquinavir (commercially available as Fortovase®), zalcitabine (commercially available as Hivid®), lopinavir/ritonavir (commercially available as Kaletra®), fosamprenavir calcium (commercially available as Lexiva®), ritonavir (commercially available as Norvir®), zidovudine (commercially available as Retrovir®), atazanavir sulfate (commercially available as Reyataz®), efavirenz (commercially available as Sustiva®), abacavir/lamivudine/zidovudine (commercially available as Trizivir®), didanosine (commercially available as Videx®), nelfinavir mesylate (commercially available as Viracept®), nevirapine (commercially available as Viramune®), tenofovir disoproxil fumarate (commercially available as Viread®), stavudine (commercially available as Zerit®), and abacavir sulfate (commercially available as Ziagen®); homocysteiene removers, including betaine anhydrous (commercially available as Cystadane®); medications, such as insulin (commercially available as Apidra®, Humalog®, Humulin®, Iletin®, and Novolin®); and HPV treatment, such as Human papillomavirus vaccine (commercially available as Gardasil®) or human papillomavirus bivalent (commercially available as Cervarix®); immunosuppressants, including cyclosporine (commercially available as Gengraf®, Neoral®, Sandimmune®, and Apo-Cyclosporine®).

Agents useful in the present invention may further include prolactin inhibitors, such as bromocriptine mesylate (commercially available as Parlodel®); medications for aiding in stress tests, such as regadenoson (commercially available as Lexiscan®); baldness medication, including finasteride (commercially available as Propecia® and Proscar®); pancreatitis treatment, such as gemfibrozil (commercially available as Lopid®); hormone medications, such as norethindrone acetate/ethinyl estradiol (commercially available as femHRT®), goserelin acetate (commercially available as Zoladex®), progesterone gel (commercially available as Prochieve®), progesterone (commercially available as Prometrium®), calcitonin-salmon (commercially available as Miacalcin®), calcitriol (commercially available as Rocaltrol®), synthroid (commercially available as Levothroid®, Levoxyl®, Unithroid®), testosterone (commercially available as Testopel®, Androderm®, Testoderm®, and AndroGel®); menopause medication, such as estradiol/norethindrone acetate (commercially available as Activella®), drospirenone/estradiol (commercially available as Angeliq®), estradiol/levonorgestrel (commercially available as Climara Pro®), estradiol/norethindrone acetate (commercially available as CombiPatch®), estradiol (commercially available as Estrasorb®, Vagifem® and EstroGel®), esterified estrogens and methyltestosterone (commercially available as Estratest®), estrogen (commercially available as Alora®, Climara®, Esclim®, Estraderm®, Vivelle®, Vivelle-Dot®), estropipate (commercially available as Ogen®), conjugated estrogens (commercially available as Premarin®), and medroxyprogesterone acetate (commercially available as Provera®); menstrual medications, including leuprolide acetate (commercially available as Lupron Depot), tranexamic acid (commercially available as Lysteda®), and norethindrone acetate (commercially available as Aygestin®); and muscle relaxants, including cyclobenzaprine hydrochloride (commercially available as Flexeril®), tizanidine (commercially available as Zanaflex®), and hyoscyamine sulfate (commercially available as Levsin®).

Agents useful herein may also include osteoporosis medications, including ibrandronate sodium (commercially available as Boniva®), risedronate (commercially available as Actonel®), raloxifene hydrochloride (commercially available as Evista®, Fortical®), and alendronate sodium (commercially available as Fosamax®); ovulation enhancers, including clomiphene citrate (commercially available as Serophene®, Clomid®, Serophene®); Paget's disease treatment, such as etidronate disodium (commercially available as Didronel®); pancreatic enzyme deficiency medications, such as pancrelipase (commercially available as Pancrease® or Zenpep®); medication for the treatment of Parkinson's disease, such as pramipexole dihydrochloride (commercially available as Mirapex®), ropinirole hydrochloride (commercially available as Requip®), carbidopa/levodopa (commercially available as Sinemet CR®), carbidopa/levodopa/entacapone (commercially available as Stalevo®), selegiline hydrochloride (commercially available as Zelapar®), rasagiline (commercially available as Azilect®), entacapone (commercially available as Comtan®), and selegiline hydrochloride (commercially available as Eldepryl®); multiple sclerosis medication, such as dalfampridine (commercially available as Ampyra®) and interferon beta-I b (commercially available as Extavia®); prostate medication, including flutamide (commercially available as Eulexin®), nilutamide (commercially available as Nilandron®), dutasteride (commercially available as Avodart®), tamsulosin hydrochloride (commercially available as Flomax®), terazosin hydrochloride (commercially available as Hytrin®), and alfuzosin hydrochloride (commercially available as UroXatral®).

Films of the present invention may further include psychiatric medications, including alprazolam (available as Niravam®, Xanax®), clozopin (available as Clozaril®), haloperidol (available as Haldol®), fluoxetine hydrochloride (available as Prozac®), sertraline hydrochloride (available as Zoloft®), asenapine (commercially available as Saphris®), iloperidone (commercially available as Fanapt®), paroxtine hydrochloride (available as Paxil®), aripiprazole (commercially aavialbe as Abilify®), guanfacine (commercially available as Intuniv®), Amphetamines and methamphetamines (commercially available as Adderall® and Desoxyn®), clomipramine hydrochloride (commercially available as Anafranil®), Buspirone hydrochloride (commercially available as BuSpar®), citalopram hydrobromide (commercially available as Celexa®), duloxetine hydrochloride (commercially available as Cymbalta®), methylphenidate (commercially available as Ritalin, Daytrana®), divalproex sodium (Valproic acid) (commercially available as Depakote®), dextroamphetamine sulfate (commercially available as Dexedrine®), venlafaxine hydrochloride (commercially available as Effexor®), selegiline (commercially available as Emsam®), carbamazepine (commercially available as Equetro®), lithium carbonate (commercially available as Eskalith®), fluvoxamine maleate/dexmethylphenidate hydrochloride (commercially available as Focalin®), ziprasidone hydrochloride (commercially available as Geodon®), ergoloid mesylates (commercially available as Hydergine®), escitalopram oxalate (commercially available as Lexapro®), chlordiazepoxide (commercially available as Librium®), molindone hydrochloride (commercially available as Moban®), phenelzine sulfate (commercially available as Nardil®), thiothixene (commercially available as Navane®), desipramine hydrochloride (commercially available as Norpramin®), benzodiazepines (such as those available as Oxazepam®), nortriptyline hydrochloride (commercially available as Pamelor®), tranylcypromine sulfate (commercially available as Parnate®), prochlorperazine, mirtazapine (commercially available as Remeron®), risperidone (commercially available as Risperdal®), quetiapine fumarate (commercially available as Seroquel®), doxepin hydrochloride (commercially available as Sinequan®), atomoxetine hydrochloride (commercially available as Strattera®), trimipramine maleate (commercially available as Surmontil®), olanzapine/fluoxetine hydrochloride (commercially available as Symbyax®), imipramine hydrochloride (commercially available as Tofranil®), protriptyline hydrochloride (commercially available as Vivactil®), bupropion hydrochloride (commercially available as Wellbutrin®, Wellbutrin SR®, and Wellbutrin XR®), and olanzapine (commercially available as Zyprexa®).

Agents useful herein may also include uric acid reduction treatment, including allopurinol (commercially available as Zyloprim®); seizure medications, including gabapentin (commercially available as Neurontin®), ethotoin (commercially available as Peganone®), vigabatrin (commercially available as Sabril®), and topiramate (commercially available as Topamax®); treatment for shingles, such as zoster vaccine live (commercially available as Zostavax®); skin care medications, including calcipotriene (commercially available as Dovonex®), ustekinumab (commercially available as Stelara®), televancin (commercially available as Vibativ®), isotretinoin (commercially available as Accutane®), hydrocortisone/iodoquinol (commercially available as Alcortin ®), sulfacetamide sodium/sulfur (commercially available as Avar®), azelaic acid (commercially available as Azelex®, Finacea®), benzoyl peroxide (commercially available as Desquam-E®), adapalene (commercially available as Differin®), fluorouracil (commercially available as Efudex®), pimecrolimus (commercially available as Elidel®), topical erythromycin (commercially available as A/T/S®, Erycette®, T-Stat®), hydrocortisone (commercially available as Cetacort®, Hytone®, Nutracort®), metronidazole (commercially available as MetroGel®), doxycycline (commercially available as Oracea®), tretinoin (commercially available as Retin-A® and Renova®), mequinol/tretinoin (commercially available as Solage®), acitretin (commercially available as Soriatane®), calcipotriene hydrate/betamethasone dipropionate (commercially available as Taclonex®), tazarotene (commercially available as Tazorac®), fluocinonide (commercially available as Vanos®), desonide (commercially available as Verdeso®), miconazole nitrate/Zinc oxide (commercially available as Vusion®), ketoconazole (commercially available as Xolegel®), and efalizumab (commercially available as Raptiva®).

Other agents useful herein may include Sleep disorder medications, including zaleplon (available as Sonata®), eszopiclone (available as Lunesta®), zolpidem tartrate (commercially available as Ambien®, Ambien CR®, Edluar®), lorazepam (commercially available as Ativan®), flurazepam hydrochloride (commercially available as Dalmane®), triazolam (commercially available as Halcion®), clonazepam (commercially available as Klonopin®), barbituates, such as Phenobarbital®), Modafinil (commercially available as Provigil®), temazepam (commercially available as Restoril®), ramelteon (commercially available as Rozerem®), clorazepate dipotassium (commercially available as Tranxene®), diazepam (commercially available as Valium®), quazepam (commercially available as Doral®), and estazolam (commercially available as ProSom®); smoking cessation medications, such as varenicline (commercially available as Chantix®), nicotine, such as Nicotrol®, and bupropion hydrochloride (commercially available as Zyban®); and steroids, including alclometasone dipropionate (commercially available as Aclovate®), betamethasone dipropionate (commercially available as Diprolene®), mometasone furoate (commercially available as Elocon®), fluticasone (commercially available as Flonase®, Flovent®, Flovent Diskus®, Flovent Rotadisk®), fluocinonide (commercially available as Lidex®), mometasone furoate monohydrate (commercially available as Nasonex®), desoximetasone (commercially available as Topicort®), clotrimazole/betamethasone dipropionate (commercially available as Lotrisone®), prednisolone acetate (commercially available as Pred Forte®, Prednisone®, Budesonide Pulmicort®, Rhinocort Aqua®), prednisolone sodium phosphate (commercially available as Pediapred®), desonide (commercially available as Tridesilon®), and halobetasol propionate (commercially available as Ultravate®).

Films of the present invention may further include agents useful for thyroid disease treatment, such as hormones TC and TD (commercially available as Armour Thyroid®); potassium deficiency treatment, including potassium chloride (commercially available as Micro-K®); triglycerides regulators, including omega-3-acid ethyl esters (commercially available as Omacor®); urinary medication, such as phenazopyridine hydrochloride (commercially available as Pyridium®) and methenamine, methylene blue/phenyl salicylate/benzoic acid/atropine sulfate/hyoscyamine (commercially available as Urised®); prenatal vitamins (commercially available as Advanced Natalcare®, Materna®, Natalins®, Prenate Advance®); weight control medication, including orlistat (commercially available as Xenical®) and sibutramine hydrochloride (commercially available as Meridia®).

The popular H₂-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts.

The pharmaceutically active agents employed in the present invention may include allergens or antigens, such as, but not limited to, plant pollens from grasses, trees, or ragweed; animal danders, which are tiny scales shed from the skin and hair of cats and other furred animals; insects, such as house dust mites, bees, and wasps; and drugs, such as penicillin.

An anti-oxidant may also be added to the film to prevent the degradation of an active, especially where the active is photosensitive.

Cosmetic active agents may include breath freshening compounds like menthol, other flavors or fragrances, especially those used for oral hygiene, as well as actives used in dental and oral cleansing such as quaternary ammonium bases. The effect of flavors may be enhanced using flavor enhancers like tartaric acid, citric acid, vanillin, or the like.

The film products are capable of accommodating a wide range of amounts of the active ingredient. The films are capable of providing an accurate dosage amount (determined by the size of the film and concentration of the active in the original polymer/water combination) regardless of whether the required dosage is high or extremely low. Therefore, depending on the type of active or pharmaceutical composition that is incorporated into the film, the active amount may be as high as about 300mg, desirably up to about 150mg or as low as the microgram range, or any amount therebetween.

### Forming the Film

The films of the present invention may be formed into a film strip or a sheet prior to drying. After the desired components are combined to form a multi-component matrix, including the polymer, water, and the active, as well as any other component as desired, the combination is formed into a sheet or film, by any method known in the art such as coating, spreading, casting or drawing the multi-component matrix. If a multi-layered film is desired, this may be accomplished by co-extruding more than one combination of components which may be of the same or different composition. A multi-layered film may also be achieved by coating, spreading, or casting a combination onto an already formed film layer.

A number of techniques may be employed in the mixing stage to prevent bubble inclusions in the final film. To provide a composition mixture with substantially no air bubble formation in the final product, anti-foaming or surface-tension reducing agents are employed. Additionally, the speed of the mixture is desirably controlled to prevent cavitation of the mixture in a manner which pulls air into the mix. Finally, air bubble reduction can further be achieved by allowing the mix to stand for a sufficient time for bubbles to escape prior to drying the film. Desirably, the inventive process first forms a masterbatch of film-forming components without active ingredients or volatile materials. In one embodiment, the active(s) are combined with smaller mixes of the masterbatch just prior to casting. Thus, the masterbatch pre-mix can be allowed to stand for a longer time without concern for instability of the active agent or other ingredients.

Although a variety of different film-forming techniques may be used, it is desirable to select a method that will provide a flexible film, such as reverse roll coating. The flexibility of the film allows for the sheets of film to be rolled and transported for storage or prior to being cut into individual dosage forms. Desirably, the films will also be self-supporting or, in other words, able to maintain their integrity and structure in the absence of a separate support. Furthermore, the films of the present invention may be selected of materials that are edible or ingestible.

### Casting or Depositing the Film Composition

The invention uses processes for making self-supporting films having a substantially uniform distribution of components. The self supporting film is particularly useful for delivery of actives as discussed herein. The processes for making the film are designed to maintain the compositional uniformity of components distributed throughout the film, which is particularly necessary when actives, such as pharmaceutical actives, are incorporated into the film. In the pharmaceutical context, it is essential that the film is compositionally uniform so that it can be divided into individual film dosage units, each dosage unit having the appropriate amount of active when administered, such that regulatory approval can be secured.

The process may further include the preliminary steps of forming a masterbatch premix of an edible water-soluble polymer and water; optionally deaerating the premix (such as by mixing); feeding a predetermining amount of the premix to at least one mixer; adding the nanoparticles to the mixer; and mixing the components to achieve a uniform distribution thereof. Thereafter, the wet film is formed and dried.

Coating or casting methods are particularly useful for the purpose of forming the films of the present invention. Specific examples include reverse roll coating, gravure coating, immersion or dip coating, metering rod or meyer bar coating, slot die or extrusion coating, gap or knife over roll coating, air knife coating, curtain coating, or combinations thereof, especially when a multi-layered film is desired.

Roll coating, or more specifically reverse roll coating, is particularly desired when forming films in accordance with the present invention. This procedure provides excellent control and uniformity of the resulting films, which is desired in the present invention. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller.

Offset Gravure is common, where the coating is deposited on an intermediate roller before transfer to the substrate.

In the simple process of immersion or dip coating, the substrate is dipped into a bath of the coating, which is normally of a low viscosity to enable the coating to run back into the bath as the substrate emerges.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is 100% solids, the process is termed "Extrusion" and in this case, the line speed is frequently much faster than the speed of the extrusion. This enables coatings to be considerably thinner than the width of the slot.

The gap or knife over roll process relies on a coating being applied to the substrate which then passes through a "gap" between a "knife" and a support roller. As the coating and substrate pass through, the excess is scraped off.

Air knife coating is where the coating is applied to the substrate and the excess is "blown off' by a powerful jet from the air knife. This procedure is useful for aqueous coatings.

In the curtain coating process, a bath with a slot in the base allows a continuous curtain of the coating to fall into the gap between two conveyors. The object to be coated is passed along the conveyor at a controlled speed and so receives the coating on its upper face. In some embodiments, the active may be deposited by a micro-drop deposition technique onto a discrete unit doses of the film. In some embodiments the active component or particles may be printed onto the surface of a film already formed, to form a discrete printed layer of active thereon.

### Drying the Film

The drying step can also be a contributing factor with regard to maintaining the uniformity of the film composition. A controlled drying process is particularly important when, in the absence of a viscosity increasing composition or a composition in which the viscosity is controlled, for example by the selection of the polymer, the components within the film may have an increased tendency to aggregate or conglomerate.

An alternative method of forming a film with an accurate dosage, that would not necessitate the controlled drying process, would be to cast the films on a predetermined well. With this method, although the components may aggregate, this will not result in the migration of the active to an adjacent dosage form, since each well may define the dosage unit per se.

One process used to make the films is described in U.S. Patent Number 7,425,292, In this process, the films are prepared by rapidly forming a visco-elastic film by applying hot air currents to the film to prevent flow migration and intermolecular forces from creating aggregates or conglomerates thereby maintaining compositional uniform distribution of components in the film; and further drying the visco-elastic film to form a self-supporting film.

The wet film forming matrix first may be fed onto the top side of a surface prior to the application of hot air currents. The wet film is desirably formed from a deaerated matrix within a time period before the active contained therein degrades. The process may further include a step of dividing the dried film into individual dosage units of equal dimensions and compositional make-up. There may be hot air currents applied to the top surface, if desired. In such embodiments, it may be desired that hot air currents be applied to the bottom surface of the film at a higher velocity than to the top surface of the film during drying. Hot air currents applied to dry the top of the films are preferably less than that which would cause surface rippling or skinning. The air current velocity is controlled such that it does not supply a sheer stress sufficient to overcome the inherent viscosity of the film forming matrix and therefore does not disturb the top surface of the film. This permits the film to sufficiently thicken in viscosity to lock-in volumetric uniformity while permitting evaporation of water through the non-skinned surface.

When a controlled or rapid drying process is used, liquid carriers are removed from the film in a manner such that the uniformity, or more specifically, the non-self-aggregating uniform heterogeneity, that is obtained in the wet film is maintained.

Desirably, the film is rapidly dried, such that a solid, visco-elastic structure is initially formed and the contents of the film are "locked in". This can take place within the first few minutes, e.g. about the first 0.5 to about 4.0 minutes of the drying process. It may be desired to limit the amount of top air flow during this initial drying stage. Controlling the drying in this manner prevents the destruction and reformation of the film's top surface, which results from conventional drying methods. This is accomplished by forming the film and placing it on the top side of a surface having top and bottom sides. Then, heat is initially applied to the bottom side of the film to provide the necessary energy to evaporate or otherwise remove the liquid carrier. The films dried in this manner dry more quickly and evenly as compared to air-dried films, or those dried by conventional drying means. In contrast to an air-dried film that dries first at the top and edges, the films dried by applying heat to the bottom dry simultaneously at the center as well as at the edges. This also prevents settling of ingredients that occurs with films dried by conventional means.

The temperature of the film forming matrix during drying is desirably about 100°C or less, desirably about 90°C or less, and most desirably about 80°C or less. The air temperature may be substantially greater than the film matrix temperature provided that no substantial deleterious effects are imparted on the film matrix or the active or active-containing component or particles. It may be desired to dry the film such that the temperature within the film is less than the boiling point of any solvent or solvents that are within the film forming matrix. Further, it is desirable that the temperature within the film forming matrix is maintained below the degradation temperature of any actives contained within the film. It is noted, however, that the temperature outside of the film may be above the temperature within the film, and in some instances may be substantially higher than the temperature within the film.

Another method of controlling the drying process, which may be used alone or in combination with other controlled methods as disclosed above includes controlling and modifying the humidity within the drying apparatus where the film is being dried. In this manner, the premature drying of the top surface of the film may be avoided.

Another method of drying tracks that previously set forth by Magoon, which is based on an interesting property of water. Although water transmits energy by conduction and convection both within and to its surroundings, water only radiates energy within and to water. Therefore, the apparatus of Magoon includes a surface onto which the fruit pulp is placed that is transparent to infrared radiation. The underside of the surface is in contact with a temperature controlled water bath. The water bath temperature is desirably controlled at a temperature slightly below the boiling temperature of water. When the wet fruit pulp is placed on the surface of the apparatus, this creates a "refractance window." This means that infrared energy is permitted to radiate through the surface only to the area on the surface occupied by the fruit pulp, and only until the fruit pulp is dry. The apparatus of Magoon provides the films of the present invention with an efficient drying time reducing the instance of aggregation of the components of the film.

The objective of the drying processes described herein is to provide a method of drying the films that avoids complications, such as the noted "rippling" effect, that are associated with conventional drying methods and which initially dry the upper surface of the film, trapping moisture inside. In conventional oven drying methods, as the moisture trapped inside subsequently evaporates, the top surface is altered by being ripped open and then reformed.

These complications are avoided by the present drying methods, and a uniform film is provided by drying the bottom surface of the film first or otherwise preventing the formation of polymer film formation (skin) on the top surface of the film prior to drying the depth of the film. This may be achieved by applying heat as described above, or alternatively by the introduction of radiation (such as controlled microwaves) to evaporate the water or other polar solvent within the film. In some embodiments, the film is rapidly dried so as to form a visco-elastic structure within the first fifteen (15) minutes of drying, desirably within the first ten (10) minutes of drying, and more particularly within the first four (4) minutes of drying. Desirably, the film is dried at such a rapid rate that any components, including the active, do not undesirably move or aggregate together. By rapidly drying the wet matrix, a substantial number of the active particles do not have time to agglomerate.

Yet alternatively, drying may be achieved by using balanced fluid flow, such as balanced air flow, where the bottom and top air flows are controlled to provide a uniform film. In such a case, the air flow directed at the top of the film should not create a condition which would cause movement of particles present in the wet film, due to forces generated by the air currents, that is, any top air flow that is present during this drying stage should be insufficient to overcome the inherent viscosity of the film surface. Additionally, any air currents directed at the bottom of the film should desirably be controlled such that the film does not lift up due to forces from the air. Uncontrolled air currents, either above or below the film, can create non-uniformity in the final film products. The humidity level of the area surrounding the top surface may also be appropriately adjusted to prevent premature closure or skinning of the polymer surface.

The present invention yields exceptionally uniform film products when attention is paid to reducing the movement and/or aggregation of the compositional components. By avoiding the introduction of and eliminating excessive air in the mixing process, selecting polymers and solvents to provide a controllable viscosity and by controllably drying the film in a rapid manner to maintain uniformity by locking-in the active-containing components, such films result. Various drying methods include those set forth in U.S. Patent Nos. 7,425,292 and 7,357,891.

The films may initially have a thickness of about 500 µm to about 1,500 µm, or about 20 mils to about 60 mils, and when dried have a thickness from about 3 µm to about 250 µm, or about 0.1 mils to about 10 mils. In some embodiments, the film product has a thickness of greater than 0.1 mils. In some other embodiments, the film product has a thickness of about 10 mils or fewer. In some further embodiments, the film product has a thickness of about 0.5 mils to about 5 mils. Desirably, the dried films will have a thickness of about 2 mils to about 8 mils, and more desirably, from about 3 mils to about 6 mils.

Another embodiment of the present invention involves the process of producing an ingestible film comprising an active component, in which the ingestible film has a predetermined dosage for an active component. The process includes the steps of: preparing an ingestible film that includes an active component; analyzing the film to determine the amount of the active component per unit weight of the film and based on the amount of the component per unit weight of the film; determining the dimensions and/or weight of the film necessary to deliver the predetermined dosage for the active component; and adjusting the dimensions and/or weight of the film into the dimensions and/or weight determined.

In accordance with the present invention, another embodiment involves determining the dimensions and/or weight of the film necessary to deliver said predetermined dosage for said active component. In some embodiments, more than one active component may be included in the film. The active components housed within the wet-casted film layers include, without limitation, food products, pharmaceutical and cosmetic actives, drugs, medicaments, antigens or allergens such as ragweed pollen, spores, microorganisms, seeds, mouthwash components, flavors, fragrances, enzymes, preservatives, sweetening agents, colorants, spices, vitamins and supplements and combinations thereof.

Another embodiment involves adjusting the dimensions and/or weight of the film into the dimensions and/or weight determined in the previous step. The adjusting may include cutting and/or coating.

Once the assay is determined the film roll would be identified to that assay and that information fed to the cutting and packaging equipment. The cutter cuts the film rolls to the proper size. Cutting the film may be accomplished by a variety of methods, such as with a knife, razor, laser, or any other suitable means for cutting a film.

In one embodiment, the size adjustment could either be length or width or both. Since the thickness is already adjusted prior to analysis only length or width of the cut would be adjusted. Using this method achieves a higher accuracy in dosing and that the Standard Deviation and of course the variance will be much smaller with this method vs. trying to control the weight of the film piece. This brings great value to the patient that receives a consistent dose with tighter tolerances.

In another embodiment, the film is provided onto a conveyor surface having top and bottom sides. The film is fed onto the top side of the surface.

In another embodiment, the film may be analyzed to determine the amount of the active component per unit weight of the film and based on the amount of the component per unit weight of the film determining the dimensions and/or weight of the film necessary to deliver the predetermined dosage for said active component; and adjusting said film into the dimensions and/or weight determined in the previous step.

In one embodiment, the drying step may be performed prior to the step of analyzing the film to determine the amount of the active component per unit weight of the film.

In another embodiment, the drying step may be performed after to the step of analyzing the film to determine the amount of the active component per unit weight of the film.

In another embodiment, the film is packaged after the step of analyzing. Desirably the analyzing step is performed at a predetermined time during the process.

The films may initially have a thickness of about 500 µm to about 1,500 µm, or about 20 mils to about 60 mils, and when dried have a thickness from about 3 µm to about 250 µm, or about 0.1mils to about 10mils. Desirably, the dried films will have a thickness of about 2 mils to about 8 mils, and more desirably, from about 3 mils to about 6 mils.

It may be desirable to test the films of the present invention for chemical and physical uniformity during the film manufacturing process. In particular, samples of the film may be removed and tested for uniformity in film components between various samples. Film thickness and over all appearance may also be checked for uniformity. Uniform films are desired, particularly for films containing pharmaceutical active components for safety and efficacy reasons.

A method for testing uniformity includes conveying a film through a manufacturing process. This process may include subjecting the film to drying processes, dividing the film into individual dosage units, and/or packaging the dosages, among others. As the film is conveyed through the manufacturing process, for example on a conveyor belt apparatus, it is cut widthwise into at least one portion. The at least one portion has opposing ends that are separate from any other film portion. For instance, if the film is a roll, it may be cut into separate sub-rolls. Cutting the film may be accomplished by a variety of methods, such as with a knife, razor, laser, or any other suitable means for cutting a film.

One embodiment involves cutting the film in order for the film to be sampled. In one embodiment, the sampling may be achieved by removing small pieces from each of the opposed ends of the portion(s), without disrupting the middle of the portion(s). Leaving the middle section intact permits the predominant portion of the film to proceed through the manufacturing process without interrupting the conformity of the film and creating sample-inducted gaps in the film. Accordingly, the concern of missing doses is alleviated as the film is further processed, e.g., packaged. Moreover, maintaining the completeness of cut portions or sub-rolls throughout the process will help to alleviate the possibility of interruptions in further film processing or packaging due to guilty control issues, for example, alarm stoppage due to notice of missing pieces.

In accordance with the present invention, analyzing the film to determine the amount of the active component per unit weight of the film may be performed using an online analyzer or by statistical sampling. The online analyzer may be an analyzer such as a beta gauge, gamma gauge, or infrared imaging. A beta and gamma gauges consists of two basic components - a source of radiation, and a radiation detector. The web to be measured is placed between the source and detector. In addition, some sort of computer is used to process the information from the detector, and convert it into a measurement. When beta or gamma gauges strike material, some of them will pass through, while others will be stopped. The thicker (or more dense) the material, the greater the chance a particle will be stopped. By measuring the ratio of the number of particles that pass through the material to the number without any material, the thickness (or weight) of the material can be determined. Infrared imaging examines absorption and transmission of photons in the infrared energy range, based on their frequency and intensity.

Alternatively, the thickness of the film can also be controlled by manual measurement during the production process to achieve the desired thickness of the film.

In one embodiment the individual piece weights of strips maybe analyzed. The analysis involves predicting flavor loss would result in the actual API content being greater than the calculated (added) amount since the flavor is included by weight in the formulation composition as-is.

If the results show non-uniformity between film samples, the samples may easily be adjusted. The films maybe adjusted by adjusting the length, width or weight of the film or combinations thereof.

In one embodiment, the adjusting step is controlled by a computer programmed to adjust the film into a predetermined dimension and/or weight. This can save time and expense because the process does not need to repeat the steps of sampling, testing throughout the manufacturing process, and testing at multiple intervals.

In another embodiment the analyzing step involves changing the predetermined dimension and/or weight to cut the film to a dimension and/or weight necessary to deliver the predetermined dosage based on the amount of the component per unit weight of said film.

The features and advantages of the present invention are more fully shown by the following examples which are provided for purposes of illustration, and are not to be construed as limiting the invention in any way.

### EXAMPLES

### Example 1

In one embodiment of the invention, an example was conducted in order to demonstrate: (i) there is a close correlation between the weight of a film piece and the uniform distribution active present in the film piece; (ii) due to this correlation, the thickness of the film can be adjusted as the flowable film matrix is cast to account for actual losses of volatile components during the drying process. Thus, the desired "label claim" can be obtained by this prediction and adjustment process. Adjustment of the thickness results in a change in weight per unit area of the film.

The following example of the present invention includes a manufacturing process that involved the preparation of a matrix of an active and other components in water. The matrix also included flavors which contain volatile components.

The list of ingredients is as follows:

**TABLE 1**

| **Component** |
|---|
| Hypromellose |
| Erythritol |
| Polyethylene Oxide |
| Active A* |
| Peppermint |
| Calcium Carbonate |
| Sucralose |
| Sodium Hydrogen Carbonate |
| Fumed Silica |
| Titanium Dioxide |
| Monoammonium Glycyrrhizinate |
| Xanthan Gum |
| Butylated Hydroxytoluene |

| |
|---|
| *an Antimetic |

The above components were combined by mixing until a uniform mixture was achieved, and then cast as a thin, uniform layer on a substrate and passed through ovens to remove the water which is regarded as a processing aid.

The films were then dried for about 4 minutes at 90°C in accordance with the methods which maintain uniformity of active content per unit area or dosage unit in the final film, as described herein, to achieve a moisture level of about 4-6%. The films exhibited a specified weight per unit area. The drying process was expected to result in the loss of solvent i.e. moisture content, as well as volatile components from the wet coated film. The evaporation of solvent was intentional, but the loss of volatile components, which are part of the dry weight formula, are considered as manufacturing losses. These losses necessitated the adjustment of the theoretical weight downward to achieve the desired potency. In these samples, film was applied at three different wet coat weights (casting weights), each of which was dried in the same manner following the methods which maintain uniformity of active content per dosage area or unit area. The casting weights were different due to the different casting thickness. Samples of dried films were then weighed and assayed.

The coat weight targets were as follows:

| Trial A | Trial B | Trial C |
|---|---|---|
| 54mg ± 2% per unit area | 57.0mg ± 2% per unit area | 60mg ± 2% per unit area (theoretical) |

The individual piece weights of strips were analyzed for each trial and are included in TABLE 2:

**Table 2. Active M Assay Values**

| 54 mg Trial | | 54 mg Trial | | 54 mg Trial | |
|---|---|---|---|---|---|
| Piece Weight (mg) | Assay (%LC) | Piece Weight (mg) | Assay (%LC) | Piece Weight (mg) | Assay (%LC) |
| 52.55 | 93.4% | 56.80 | 101.5% | 57.45 | 102.6% |
| 52.18 | 93.0% | 56.05 | 99.6% | 58.63 | 104.4% |
| 53.27 | 94.6% | 56.81 | 97.2% | 58.71 | 104.6% |
| 53.10 | 94.3% | 56.03 | 98.9% | 58.71 | 104.3% |
| 54.85 | 97.4% | 54.60 | 101.0% | 60.28 | 107.0% |
| 54.52 | 96.9% | 55.37 | 99.7% | 59.43 | 105.8% |
| 54.78 | 97.2% | 51.39 | 98.8% | 59.97 | 106.8% |
| 54.49 | 96.9% | 55.42 | 100.3% | 59.25 | 105.6% |
| 53.24 | 95.0% | 55.92 | 99.7% | 58.86 | 104.9% |
| 52.98 | 94.3% | 55.38 | 98.8% | 58.26 | 103.6% |
| 53.44 | 95.0% | 55.85 | 99.6% | 58.81 | 104.3% |
| 53.50 | 95.2% | 55.63 | 99.1% | 58.69 | 104.7% |

The assay data were plotted versus the in weight of individual strip samples from various locations of the web. It was predicted that significant amounts of flavor loss would result in the actual active content per unit dose being greater than the amount calculated from the formula since the flavor is included by weight in the formulation composition as-is. The theoretical trial represents the weight if no volatile or solvent is lost.

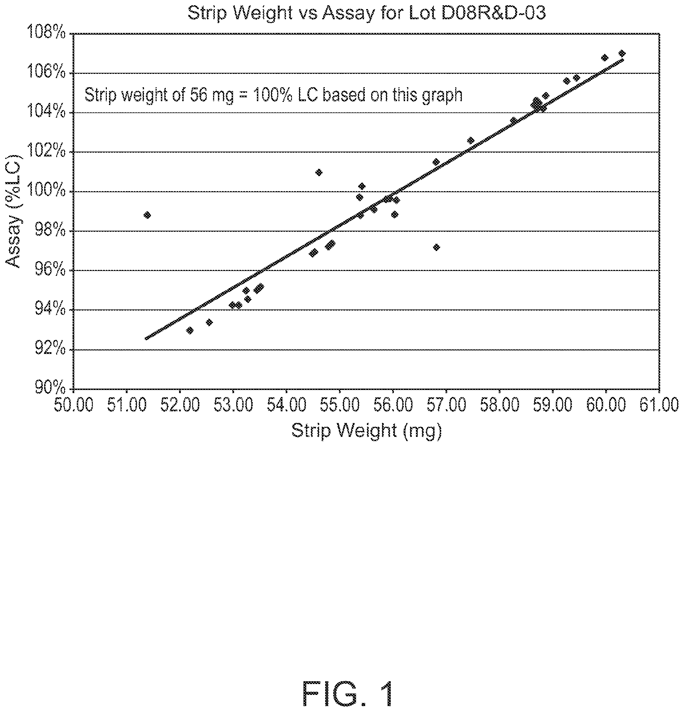

Figure 1 shows that there was excellent linear correlation between the assays at different strip weights for the active drug and the piece weight. Therefore, this indicates that one method to account for loss of volatiles during manufacturing is to adjust the piece weight by adjusting the casting thickness in order to obtain the desired amount of active in each film piece (dosage unit).

The data in Figure 1 also suggested that close to 100% label claim ("LC") for the active drug could be obtained by adjusting the strip weight downward to about 56 mg.

### Example 2

In another embodiment of the invention, an example was conducted in order to demonstrate: (i) there is a close correlation between the weight of a film piece and the uniform distribution active present in the film piece; (ii) due to this correlation, the thickness of the film can be adjusted as the flowable film matrix is cast to account for actual losses of volatile components during the drying process. Thus, the desired "label claim" can be obtained by this prediction and adjustment process. Adjustment of the thickness results in a change in weight per unit area of the film.

In the following example, similar to Example 1, a coating solution (matrix), including two actives was prepared and cast onto a substrate using the following ingredients:

**TABLE 3**

| Components |
|---|
| Polyethylene Oxide |
| Active M* |
| Maltitol Syrup |
| Natural Lime Flavor |
| Citric Acid |
| Active N*¹ |
| Hypermellose |
| Acesulfame K |
| Sodium Citrate |
| FD&C Yellow #6 Granular |

| |
|---|
| * an Opiod *¹ an Opiod Antagonist |

The above components were combined by mixing until a uniform mixture was achieved, and then cast as a thin, uniform layer on a substrate and passed through ovens to remove the water which is regarded as a processing aid.

The films were then dried for about 4 minutes at 100°C in accordance with the methods which maintain uniformity of active content per unit area or dosage unit in the final film, as described herein, to achieve a moisture level of about 3-6%.The films exhibited a specified weight per unit area. The drying process was expected to result in the loss of solvent i.e. moisture content, as well as volatile components from the wet coated film. The evaporation of solvent was intentional, but the loss of other volatile components, are considered as manufacturing losses. These losses necessitated the adjustment of the theoretical weight downward to achieve the desired potency. In these samples, film was applied at three different wet coat weights (casting weights), each of which was dried in the same manner following the methods which maintain uniformity of active content per dosage area or unit area. The casting weights were different due to the different casting thickness. Samples of dried films were then weighed.

The coat weight targets were as follows:

| Trial A | Trial B | Trial C |
|---|---|---|
| 40.0mg ± 0.5mg per unit area | 39.0 mg ± 0.5mg per unit area | 38.0mg ± 0.5mg per unit area |

After preparing the sample, the sample was analyzed to determine the amount of active present in each dosage. Several die cuts were taken from the web. Two die cuts were taken from the beginning and end of each portion for each coat weight trial. (six die cuts in total). However, the unit pieces taken from each die cut were from different parts of the sample piece as shown in Table 4 below.

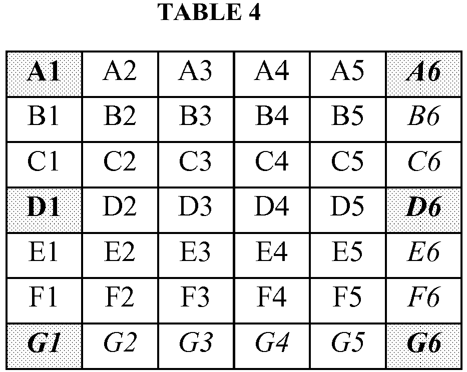

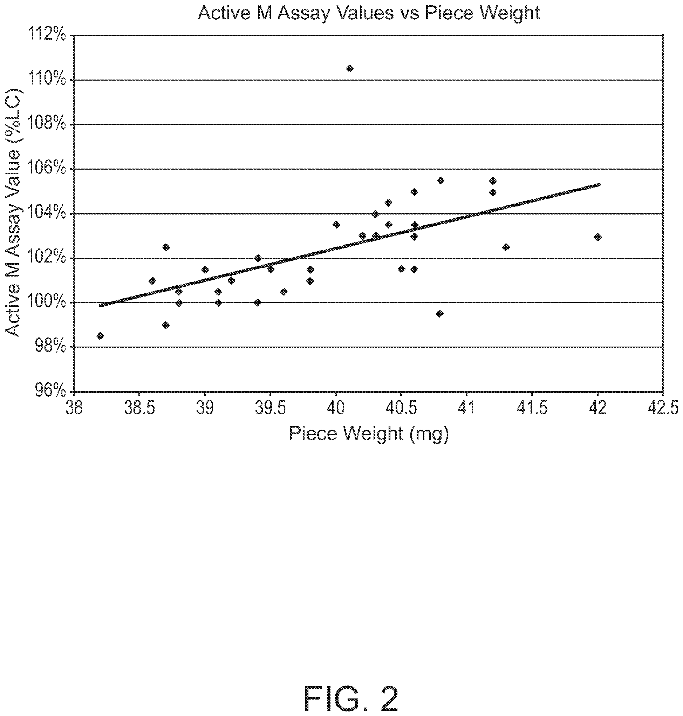

The individual film pieces (A1, D1, G1 and A6, D6, G6) shown in TABLE 3 were assayed for active M using the analytical method for individual strip assay content. This sampling resulted in a total of 12 assay values from each coat weight trial - four each from the beginning, middle and end of each coat weight trial. The resulting assay values are shown in Table 5 and are plotted against the piece weight in Figure 2.

**TABLE 5**

| **Active M Assay Values** | | | | | | |
|---|---|---|---|---|---|---|
| | **Trial A** | | **Trial B** | | **Trial C** | |
| **Location** | **% LC** | **Weight** | **% LC** | **Weight** | **% LC** | **Weight** |
| Beg | 105.5 | 41.2 | 103.5 | 40 | 103 | 40.2 |
| Beg | 105 | 41.2 | 101.5 | 39 | 101 | 39.2 |
| Beg | 101.5 | 40.5 | 100 | 39.4 | 101 | 38.6 |
| Beg | 103.5 | 40.6 | 103 | 40.3 | 102.5 | 38.7 |
| Mid | 101.5 | 40.6 | 103 | 40.6 | 100.5 | 39.6 |
| Mid | 103 | 42 | 101 | 39.8 | 99 | 38.7 |
| Mid | 99.5 | 40.8 | 105.5 | 40.8 | 100.5 | 38.8 |
| Mid | 102.5 | 41.3 | 101.5 | 39.8 | 98.5 | 38.2 |
| End | 103 | 40.6 | 103.5 | 40.4 | 101.5 | 39.5 |
| End | 105 | 40.6 | 104 | 40.3 | 100 | 38.8 |
| End | 104.5 | 40.4 | 102 | 39.4 | 100 | 39.1 |
| End | 110.5 | 40.1 | 103.5 | 40 | 100.5 | 39.1 |
| **Average** | 103.75 | 40.83 | 102.67 | 39.99 | 100.67 | 39.03 |
| **StdDev** | 2.75 | 0.51 | 1.51 | 0.53 | 1.29 | 0.54 |
| **% RSD** | 2.65 | 1.24 | 1.47 | 1.33 | 1.28 | 1.39 |

### Results and Conclusions

Figure 2 shows that there was excellent linear correlation between the assays at different strip weights for the active drug. Therefore, this indicates that one method to account for loss of volatiles during manufacturing is to adjust the piece weight by adjusting the thickness of the cast film can be used as a means to adjust and obtain the desired amount of active in each film piece (dosage unit).

On the basis of the data for the active M assay, a 39 mg piece weight can be used to compensate for the loss of flavor from the formulation and maintain the proper quantity of active required (label claim). To simplify documentation and production, it is customary to round up the piece weight to the nearest whole mg. From the chart, the piece weight associated with 100% LC is approximately 38.4 mg. In order to assure the product is at least 100% LC, that value is rounded up to 39mg.

### Example 3

In another embodiment of the invention, an example was conducted in order to demonstrate: (i) there is a close correlation between the weight of a film piece and the uniform distribution active present in the film piece; (ii) due to this correlation, the length of the film can be adjusted as the film matrix is cut to achieve the desired target assay. Thus, the desired "label claim" can be obtained by this prediction and adjustment process. Adjustment of the length results in a change in the weight of the unit dose.

The components provided in Example 2, were combined by mixing until a uniform mixture was achieved, and then cast as a thin, uniform layer on a substrate and passed through ovens to remove the water which is regarded as a processing aid.

The films were then dried for about 4 minutes at 100°C in accordance with the methods which maintain uniformity of active content per unit area or dosage unit in the final film, as described herein, to achieve a moisture level of about 2-4%. The films exhibited a specified weight per unit area. The drying process was expected to result in the loss of solvent i.e. moisture content, as well as volatile components from the wet coated film. The evaporation of solvent was intentional, but the loss of volatile components, which are part of the dry weight formula, are considered as manufacturing losses.

In this Example, the film was analyzed using an offline technique. In particular the offline technique involved cutting the length of the strip in order to achieve a desired active content.

The process involved analyzing 8 samples as shown below in Table 6.

**TABLE 6**

| M07XX1 In process Film Strip | | | | | | |
|---|---|---|---|---|---|---|
| | Strip Dimensions | | | | % Label | |
| Sample # | Length (mm) | Width (mm) | area (sq mm) | | Sample # | Active M |
| 1 | 24.99 | 22.23 | 555.48 | | 1 | 96.28 |
| 2 | 24.61 | 21.82 | 537.01 | | 2 | 95.99 |
| 3 | 24.99 | 22.23 | 555.48 | | 3 | 96.11 |
| 4 | 24.99 | 22.23 | 555.48 | | 4 | 95.68 |
| 5 | 24.61 | 21.82 | 537.01 | | 5 | 96.97 |
| 6 | 24.99 | 21.82 | 545.33 | | 6 | 89.77 |
| 7 | 24.99 | 21.82 | 545.33 | | 7 | 94.9 |
| 8 | 24.99 | | 555.48 | | 8 | 93.58 |
| Average | 24.90 | 22.23 22.02 | 548.33 | | Average | 94.91 |
| Theory | 25.40 | 22.00 | 545.33 | | Adjusted to length 25.4 mm | 96.82 |
| Actual length as % of Theory | | | 103.28% | | Adjusted to length 26.2 mm | 100.00 |

The above data indicates that the in-process assay values for the 8 samples were on the average 94.91 % label claim ("LC") for active M. Measured dimensions however indicate the average length was 24.9 mm, short of the target 25.4 mm.

The average assay values were estimated based on a full size piece of 25.4 mm gives 96.82 %LC for active M. Accordingly, it was predicted that in order to achieve an assay as close to 100% as possible and stay within the product specification, the strip length should be adjusted to 26.3 mm which should give assay values of 100.00 for active M.

The results indicate that one method to achieve the desired target assay during manufacturing is to adjust the piece weight by adjusting the length of the cast film can be used as a means to adjust and obtain the desired amount of active in each film piece (dosage unit).

### Example 4

In another embodiment of the invention, an example was conducted in order to demonstrate that if a continuous measurement of the active is available on line, the thickness of the film can be adjusted to provide the desired %LC.

The following example of the present invention includes a manufacturing process that involved the preparation of a solution/suspension of the drug and other components in water to form a matrix. The solution/suspension also included flavors which contain volatile components. In this Example, the film was analyzed using an online technique. In particular the online technique involved Near-Infrared (NIR) radiation.

In the following example, a coating solution (matrix), was prepared and cast onto a substrate using the following ingredients:

**TABLE 7**

| **Components** |
|---|
| Hypermellose |
| Active D* |
| Propylene Glycol |
| Hupermellose |
| Polyethylene Oxide |
| Polydextrose |
| Glycerine |
| Sucralose |
| Propylene Glycol Alginate |
| Glyceryl Monostearate |
| Precipitated Silica |
| Hydrophilic TiO2 |
| Magnesium Stearate |
| Methyl Paraben |

| |
|---|
| *a Flavor Component |

The above components were combined by mixing until a uniform mixture was achieved, and then cast as a thin, uniform layer on a substrate and passed through ovens to remove the water which is regarded as a processing aid.

The films were then dried for about 3 minutes at 120°C in accordance with the methods which maintain uniformity of active content per unit area or dosage unit in the final film, as described herein, to achieve a desired moisture level of about 3-6%. The films exhibited a specified weight per unit area. The drying process was expected to result in the loss of solvent i.e. moisture content, as well as volatile components from the wet coated film. The evaporation of solvent was intentional, but the loss of volatile components, which are part of the dry weight formula, are considered as manufacturing losses.

NIR radiation was applied to the dried film in order to measure the drug content per unit area. The IR Spectra measures the absorbance of the active per unit area, which provides a spectra indicative of the amount of active present per unit area.

Continuous monitoring of the NIR Spectra was required in order to identify the loss of volatiles and thereby the need for an adjustment of the thickness of the weight per unit area of the film.

### Results and conclusions:

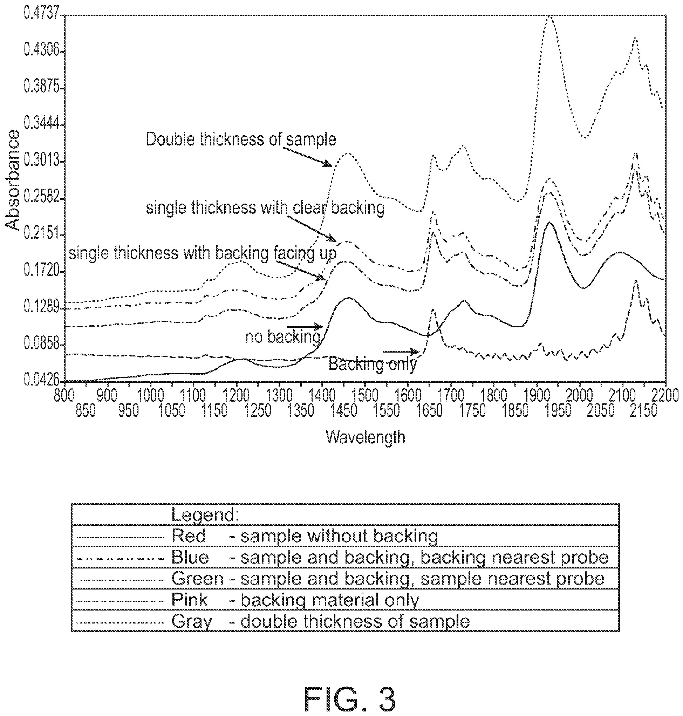

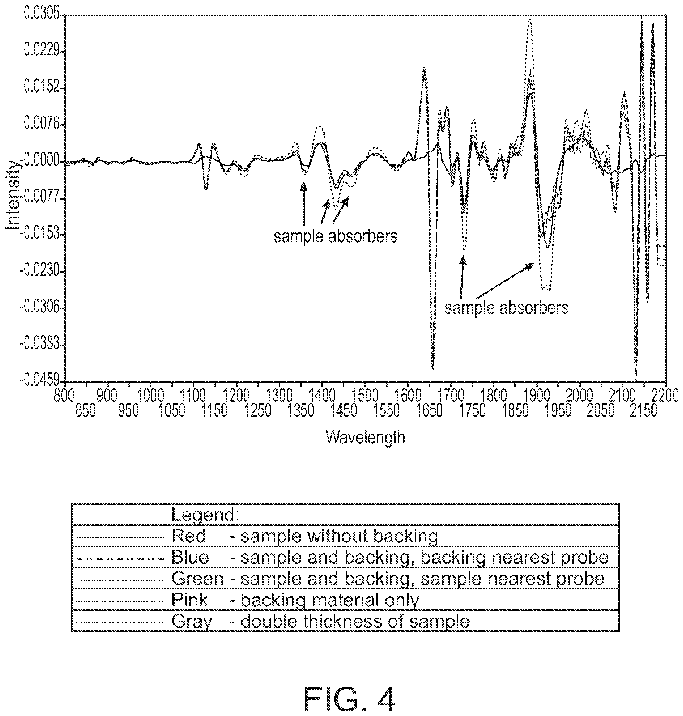

Figures 3 and 4 show the absorbance spectra for Example 4. In particular, Figure 4 shows the second derivative absorbance spectra. By converting to second derivative, data, more detail is obtained from the spectra. The arrows on Figure 4 show the 2nd derivative peaks where the sample has a strong 2nd derivative, with no effect from the backing. The gray peak is larger, indicating higher sensitivity due to the doubling of the sample thickness.

Monitoring these absorbance peaks at 9 points across the web continuously using 9 sensors gave continuous measurement of the active concentration both across the web and along the web. Alternatively, the web can be scanned by a single sensor to obtain comparable data.

This can be used when multiple sub-lots are manufactured with the active being added to smaller mixers with portions of the master batch. In that case, the measurement assures that the mix was done correctly and that the drug concentration is consistent sub-lot to sub-lot.

In another embodiment, the sensors can be used in a feedback control loop where they can control the gap on the coating head and subsequently the coat weight.

The results indicate that by taking a series of readings of NIR spectra, a calculation can be made to show the need to adjust the film dimensions, as a means to adjust the thickness in order to obtain the desired amount of active in each film piece (dosage unit).

### Example 5

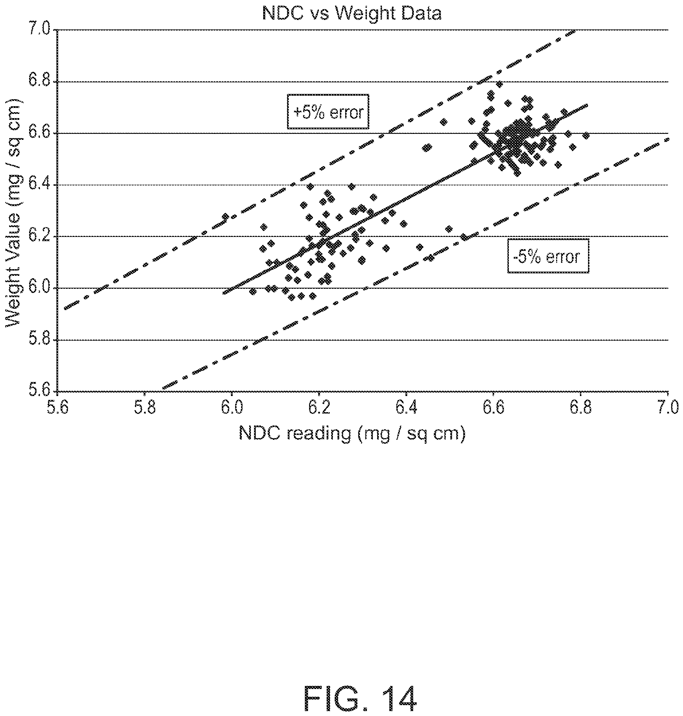

In another embodiment of the invention, an example was conducted in order to demonstrate the ability to control coat weight and subsequently the active concentration per unit area in a film by adjusting the thickness of the film using the NDC gamma gauge ("NDC"). By taking a series of readings using a sensitive gauge, a calculation can be made to show if it is necessary to adjust the film dimensions. Then a prediction and adjustment of the film dimensions can be made in order compensate for manufacturing losses as shown in Figure 14. A feedback loop can be used to provide the adjustment information to the manufacturing machinery.

The following example of the present invention includes a manufacturing process that involved the preparation of a matrix of the active and other components in water. The solution/suspension also included flavors which contain volatile components.

The films were then dried for about 3 minutes at 120°C in accordance with the methods which maintains uniformity of active content in the final film, as per dosage unit or area described herein to a moisture level of about 3-7%. The films exhibited a specified weight per unit area. The drying process was expected to result in the loss of solvent i.e. moisture content as well as volatile components from the wet coated film. The evaporation of solvent was intentional, but the loss of volatile components, which are part of the dry weight formula, are considered as manufacturing losses.

In this Example, the film was analyzed using an online technique. In particular the online technique demonstrates the accuracy of coat weight control using an NDC gamma gauge.

For these batches, the NDC gamma gauge was used to control the gap on the coating head, and thus the coat weight. Data comparing actual coat weight with the NDC measurement set point were compiled and evaluated.

The data was obtained from 4 batches of Product E, 4 batches of product F and one batch of Product G. The data is contained in the following tables. All values are in mg / sq cm.

The coating solution for Product E was composed of the following:

**TABLE 8**

| **Components** |
|---|
| Hypermellose |
| Active E* |
| Polydextrose |
| Polyethylene Oxide |
| Propylene Glycol |
| Glycerin |
| Sucralose |
| Propylene Glycol Alginate |
| Glyceryl Monostearate |
| Precipitated Silica |
| Titanium Dioxide |
| Magnesium Stearate |
| Methylparaben |

| |
|---|
| *a Flavor Component |

Four batches of the solution were coated onto a mylar substrate and 2 X 50 sq cm samples taken periodically, weighed and the data recorded as Actual. Data comparing actual coat weight to control limits were compiled and evaluated. The data were obtained from 4 batches of Product E, 4 batches of product F and one batch of Product G. The data consists of measured coat weight for 3 locations (2 edges and middle) on the web, designated as Operator, Middle and Machine. The data are contained in TABLE 9. All values are in mg / sq cm

**TABLE 9**

| **Batch A** | | | **Batch B** | | | **Batch C** | | | **Batch D** | | |
|---|---|---|---|---|---|---|---|---|---|---|---|
| **Operator** | **Middle** | **Drive** | **Operator** | **Middle** | **Drive** | **Operator** | **Middle** | **Drive** | **Operator** | **Middle** | **Drive** |
| 6.385 | 6.580 | 6.904 | 6.590 | 6.500 | 6.630 | 6.620 | 6.504 | 6.667 | 6.529 | 6.523 | 6.726 |
| 6.435 | 6.505 | 6.717 | 6.638 | 6.570 | 6.631 | 6.567 | 6.515 | 6.658 | 6.520 | 6.565 | 6.622 |
| 6.383 | 6.541 | 6.791 | 6.719 | 6.661 | 6.737 | 6.583 | 6.539 | 6.722 | 6.589 | 6.507 | 6.584 |
| 6.451 | 6.523 | 6.734 | 6.651 | 6.553 | 6.639 | 6.587 | 6.584 | 6.759 | 6.712 | 6.505 | 6.596 |
| 6.486 | 6.550 | 6.802 | 6.639 | 6.544 | 6.635 | 6.574 | 6.559 | 6.686 | 6.491 | 6.433 | 6.586 |
| 6.381 | 6.530 | 6.743 | 6.667 | 6.594 | 6.716 | 6.561 | 6.557 | 6.709 | 6.579 | 6.434 | 6.545 |
| 6.395 | 6.423 | 6.667 | 6.558 | 6.436 | 6.518 | 6.521 | 6.547 | 6.747 | 6.552 | 6.490 | 6.464 |
| 6.427 | 6.448 | 6.683 | 6.482 | 6.420 | 6.492 | 6.519 | 6.506 | 6.599 | 6.744 | 6.603 | 6.738 |
| 6.506 | 6.561 | 6.724 | 6.574 | 6.537 | 6.570 | 6.569 | 6.512 | 6.671 | 6.693 | 6.428 | 6.477 |
| 6.444 | 6.518 | 6.665 | 6.644 | 6.579 | 6.706 | 6.651 | 6.603 | 6.800 | 6.597 | 6.366 | 6.483 |
| 6.482 | 6.543 | 6.773 | 6.624 | 6.529 | 6.655 | 6.590 | 6.534 | 6.666 | 6.863 | 6.727 | 6.782 |
| 6.461 | 6.470 | 6.710 | 6.655 | 6.538 | 6.618 | 6.598 | 6.471 | 6.563 | 6.500 | 6.415 | 6.545 |
| 6.688 | 6.502 | 6.468 | 6.615 | 6.585 | 6.633 | 6.621 | 6.582 | 6.669 | 6.547 | 6.430 | 6.503 |
| 6.561 | 6.540 | 6.661 | 6.501 | 6.433 | 6.476 | 6.608 | 6.439 | 6.466 | 6.566 | 6.461 | 6.533 |
| 6.653 | 6.608 | 6.648 | 6.605 | 6.548 | 6.587 | 6.478 | 6.418 | 6.539 | 6.505 | 6.436 | 6.528 |
| 6.621 | 6.601 | 6.714 | 6.590 | 6.549 | 6.648 | 6.618 | 6.657 | 6.617 | 6.701 | 6.530 | 6.601 |
| 6.591 | 6.529 | 6.534 | 6.617 | 6.566 | 6.601 | 6.615 | 6.509 | 6.648 | 6.604 | 6.544 | 6.693 |
| 6.638 | 6.582 | 6.656 | 6.558 | 6.509 | 6.577 | 6.548 | 6.554 | 6.751 | 6.472 | 6.489 | 6.530 |
| 6.609 | 6.562 | 6.611 | 6.705 | 6.585 | 6.579 | 6.574 | 6.523 | 6.692 | 6.494 | 6.442 | 6.555 |
| 6.554 | 6.545 | 6.609 | 6.669 | 6.652 | 6.614 | 6.607 | 6.594 | 6.762 | 6.551 | 6.505 | 6.702 |
| 6.623 | 6.601 | 6.665 | | | | 6.605 | 6.585 | 6.811 | 6.625 | 6.500 | 6.605 |
| 6.691 | 6.625 | 6.841 | | | | | | | 6.555 | 6.491 | 6.626 |
| 6.596 | 6.555 | 6.606 | | | | | | | 6.568 | 6.431 | 6.495 |
| 6.660 | 6.534 | 6.648 | | | | | | | | | |

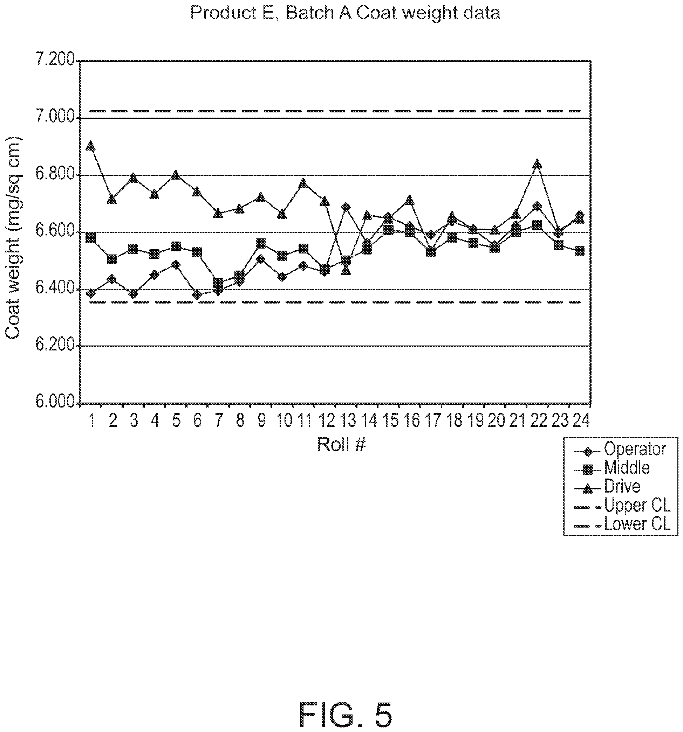

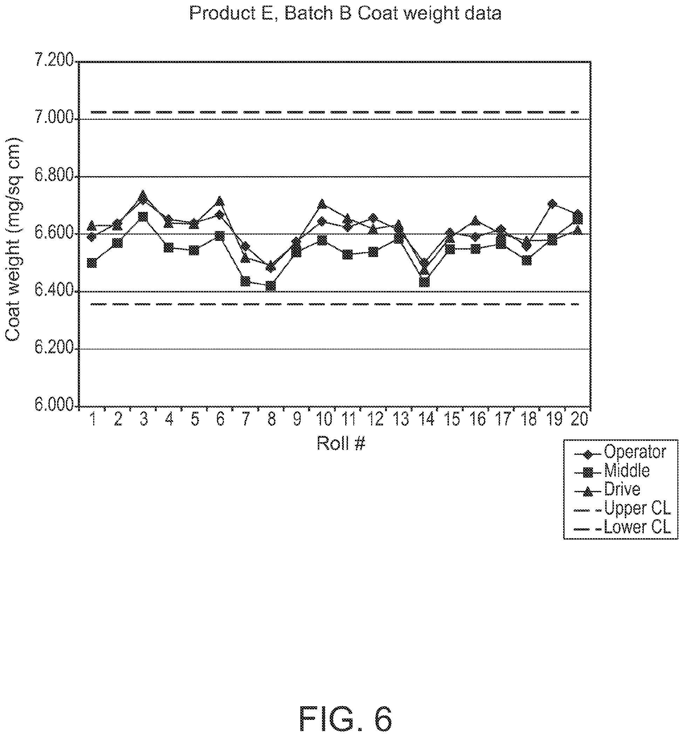

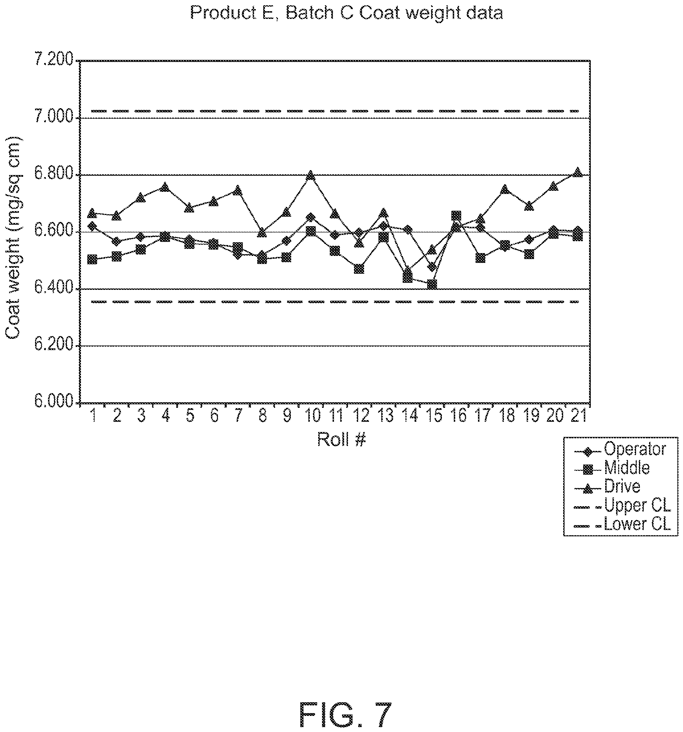

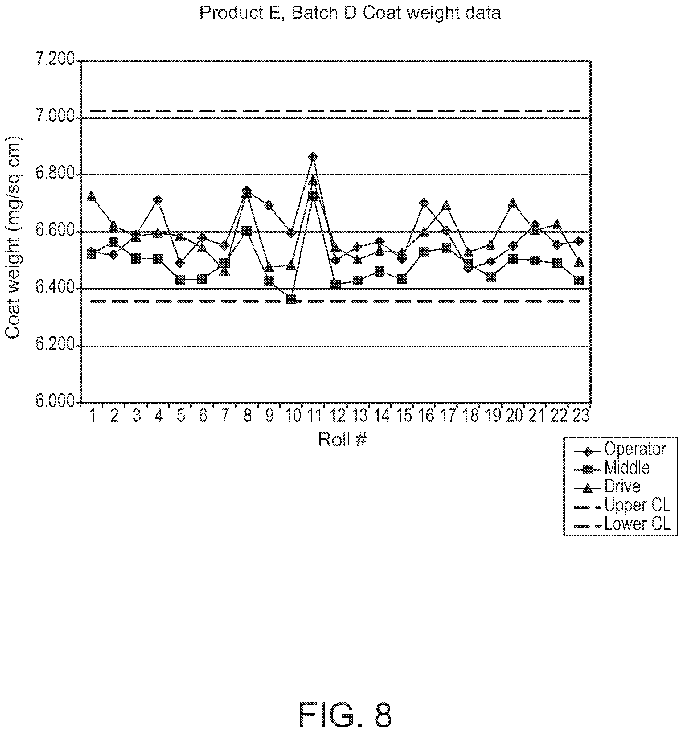

To demonstrate that the NDC is controlling within the control limits, the actual measured values are plotted along with the control limits in the 4 plots, as shown in Figures 5-8

The coating solution for Product F was composed of the following:

**TABLE 10**

| |
|---|
| Components |
| Hypermellose |
| Active F* |
| Polydextrose |
| Polyethylene Oxide |
| Propylene Glycol |
| Glycerin |
| Sucralose |
| Glyceryl Monostearate |
| Precipitated Silica |
| Titanium Dioxide |
| Methylparaben |

| |
|---|
| *Flavor component |

Four batches of the solution were cast onto a Mylar substrate and 2 x 50 sq cm samples taken at the 3 locations at the end of each finished roll, weighed and the data recorded as Actual. The data for all 3 locations for the 4 batches is recorded in the following table.

**TABLE 11**

| **Batch E** | | | | | | **Batch G** | | | **Batch H** | | |
|---|---|---|---|---|---|---|---|---|---|---|---|
| **operator** | **Middle** | **Drive** | **Operator** | **Middle** | **Drive** | **Operator** | **Middle** | **Drive** | **Operator** | **Middle** | **Drive** |
| 6.030 | 6.029 | 6.205 | 6.214 | 6.131 | 6.151 | 6.078 | 6.129 | 6.187 | 6.275 | 6.227 | 6.100 |
| 6.138 | 6.141 | 6.203 | 6.211 | 6.212 | 6.223 | 5.923 | 6.136 | 6.170 | 5.907 | 6.031 | 5.907 |
| 6.163 | 6.099 | 6.163 | 6.191 | 6.201 | 6.292 | 5.932 | 6.149 | 6.115 | 5.939 | 6.259 | 5.982 |
| 6.175 | 6.146 | 6.192 | 6.217 | 6.300 | 6.222 | 5.902 | 6.075 | 6.049 | 6.388 | 6.299 | 6.431 |
| 6.174 | 6.164 | 6.208 | 6.154 | 6.337 | 6.474 | 5.942 | 6.124 | 6.075 | 6.390 | 6.199 | 6.457 |
| 6.285 | 6.245 | 6.330 | 6.311 | 6.378 | 6.494 | 5.982 | 6.110 | 6.030 | 6.397 | 6.224 | 6.366 |
| 6.236 | 6.234 | 6.268 | 6.179 | 6.112 | 6.164 | 6.089 | 6.140 | 6.141 | 5.969 | 6.412 | 6.190 |
| 6.102 | 6.132 | 6.180 | | | | 6.188 | 6.144 | 6.076 | 6.021 | 6.329 | 6.234 |
| 6.284 | 6.232 | 6.310 | | | | 6.195 | 6.145 | 6.016 | 6.053 | 6.260 | 6.157 |
| 6.243 | 6.254 | 6.328 | | | | 6.009 | 6.098 | 6.026 | 5.936 | 6.290 | 6.077 |
| 6.213 | 6.173 | 6.192 | | | | 6.091 | 6.177 | 6.082 | 6.015 | 6.196 | 6.091 |
| 6.340 | 6.254 | 6.228 | | | | 6.190 | 6.163 | 6.019 | 6.054 | 6.262 | 6.134 |
| 6.294 | 6.335 | 6.294 | | | | 6.074 | 6.200 | 6.064 | 6.091 | 6.434 | 6.123 |
| 6.222 | 6.394 | 6.284 | | | | 5.902 | 6.094 | 5.907 | 6.121 | 6.332 | 6.273 |
| 6.269 | 6.393 | 6.344 | | | | 6.046 | 6.102 | 6.095 | 5.969 | 6.356 | 6.096 |
| 6.321 | 6.401 | 6.459 | | | | 5.902 | 6.091 | 5.978 | 5.980 | 6.262 | 6.130 |
| 6.119 | 6.459 | 6.129 | | | | 6.125 | 6.081 | 5.951 | 6.087 | 6.375 | 6.108 |
| 6.308 | 6.336 | 6.460 | | | | 5.896 | 6.030 | 6.033 | 6.058 | 6.359 | 6.156 |
| 6.181 | 6.319 | 6.392 | | | | 5.954 | 6.103 | 5.919 | 6.145 | 6.441 | 6.217 |
| 6.072 | 5.985 | 5.909 | | | | 5.947 | 5.989 | 6.000 | 6.014 | 6.391 | 6.119 |
| 6.145 | 6.142 | 6.229 | | | | 6.058 | 6.168 | 6.092 | | | |
| 6.248 | 6.317 | 6.495 | | | | 5.991 | 6.184 | 6.07 | | | |
| | | | | | | 6.049 | 6.15 | 6.089 | | | |
| | | | | | | 6.105 | 6.187 | 6.174 | | | |

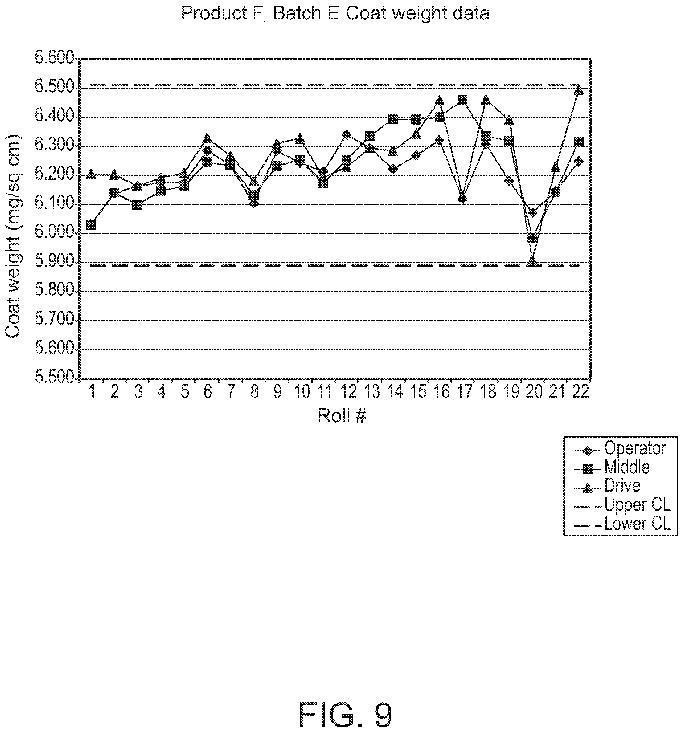

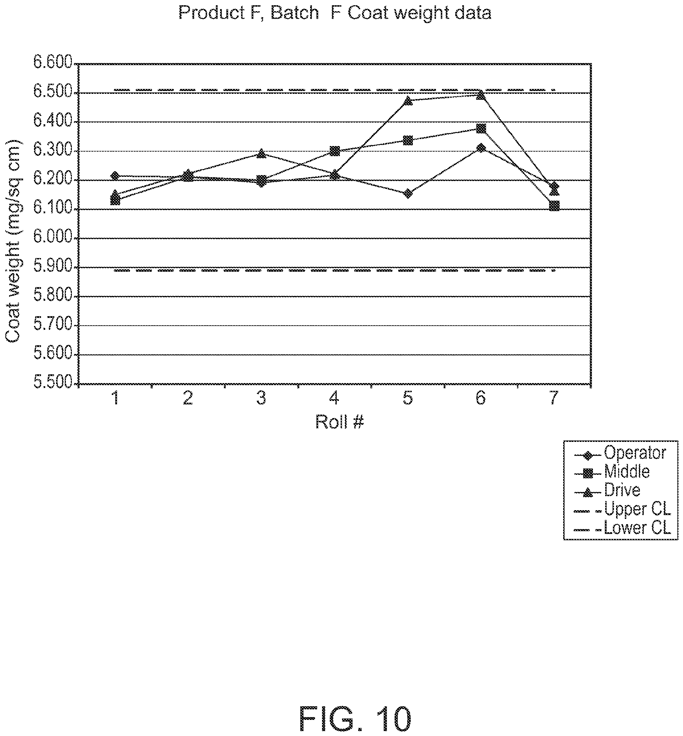

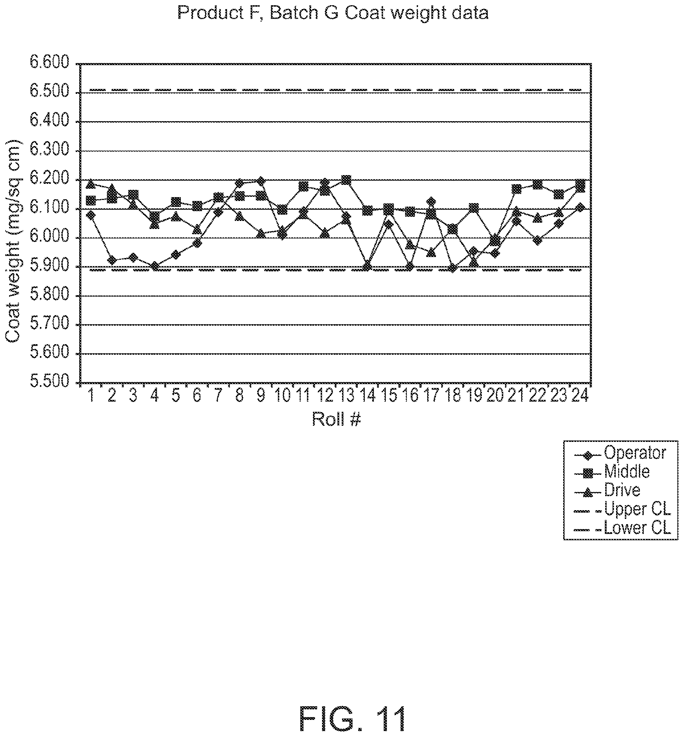

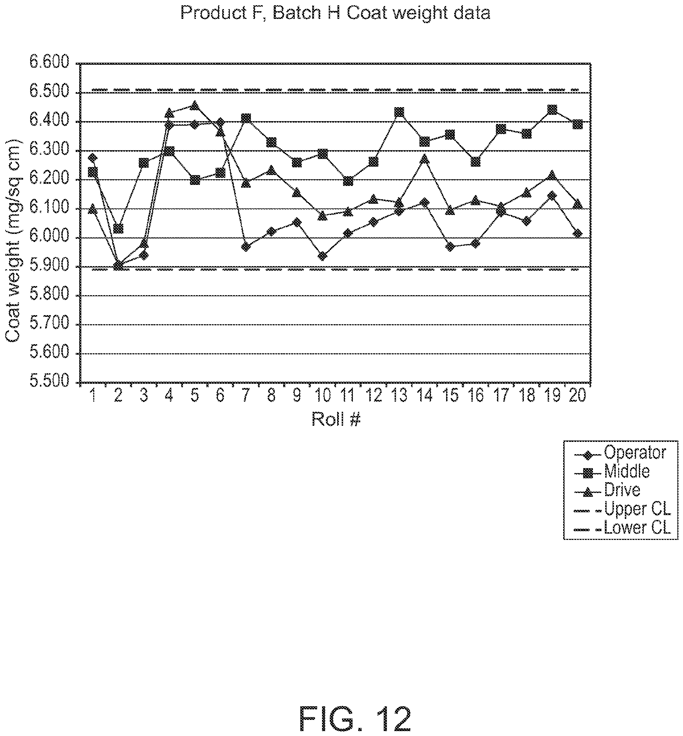

To demonstrate that the NDC is controlling within the control limits, the actual measured values are plotted along with the control limits in the 4 plots, as shown in Figures 9 - 12.

The coating solution for Product G was composed of the following:

**TABLE 12**

| **Components** |
|---|
| Hypermellose |
| Active F |
| Polydextrose |
| Polyethylene Oxide |
| Propylene Glycol |
| Glycerin |
| Sucralose |
| Propylene Glycol Alginate |
| Glyceryl Monostearate |
| Precipitated Silica |
| Titanium Dioxide |
| Magnesium Stearate |
| Methyl Paraben |

| |
|---|
| *Flavor component |

The solution was cast onto a mylar substrate and 2 x 50 sq cm samples taken at the 3 locations at the end of each finished roll, weighed and the data recorded as Actual. The data for all 3 locations is recorded in the following table.

**Table 13**

| **Batch I** | | |
|---|---|---|
| **Operator** | **Middle** | **Drive** |
| 6.6 | 6.7 | 6.7 |
| 6.6 | 6.7 | 6.7 |
| 6.7 | 6.6 | 6.5 |
| 6.7 | 6.5 | 6.7 |
| 6.5 | 6.5 | 6.6 |
| 6.5 | 6.5 | 6.5 |
| 6.6 | 6.5 | 6.7 |
| 6.6 | 6.5 | 6.6 |
| 6.7 | 6.5 | 6.8 |
| 6.6 | 6.6 | 6.6 |
| 6.7 | 6.7 | 6.8 |
| 6.6 | 6.6 | 6.7 |
| 6.7 | 6.6 | 6.7 |
| 6.7 | 6.6 | 6.7 |
| 6.7 | 6.6 | 6.7 |
| 6.6 | 6.4 | 6.6 |
| 6.6 | 6.5 | 6.6 |
| 6.7 | 6.5 | 6.7 |
| 6.5 | 6.5 | 6.6 |
| 6.7 | 6.7 | 6.8 |
| 6.8 | 6.6 | 6.8 |
| 6.6 | 6.5 | 6.8 |
| 6.6 | 6.6 | 6.7 |
| 6.7 | 6.7 | 6.8 |

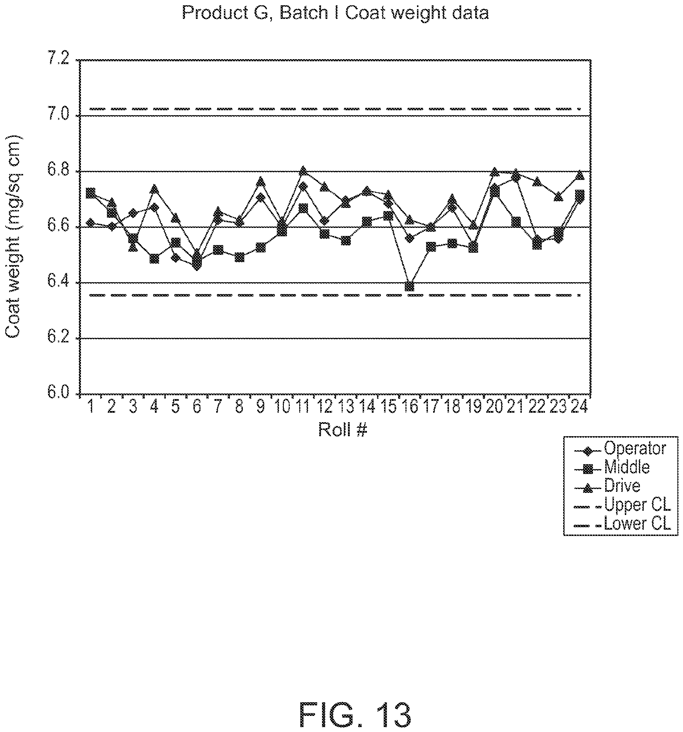

To demonstrate that the NDC is controlling within the control limits, the actual measured values are plotted along with the control limits as shown in Figure 13

### Results and conclusions:

The results indicate that by taking a series of readings using a sensitive gauge i.e gamma gauge, a calculation can be made to show the need to adjust the film dimensions, as a means to adjust the thickness of the film in order to obtain the desired amount of active in each film piece (dosage unit) as shown in Figure 14.

## Claims

1. A process for producing an ingestible film comprising an active component, said ingestible film having a predetermined dosage for said active component, said process comprising:
a) preparing a continuous film comprising a polymer component and said active component uniformly dispersed in said polymer component;
b) analyzing said film to determine the amount of said active component per unit weight of said film and based on said amount of said component per unit weight of said film determining a dimension of the film necessary to deliver said predetermined dosage for said active component;
c) adjusting said dimension of the film into the dimension determined in step (b).

2. The process of claim 1, wherein said active component is selected from the group consisting of: food products, pharmaceutical agents, cosmetic agents, drugs; medicaments; antidotes; vaccines; antigens or allergens; mouthwash components; flavors; fragrances; enzymes; preservatives; sweetening agents; colorants; spices; vitamins; cooling agents; tingling agents, and combinations thereof.

3. The process of claim 1 , wherein said polymer component comprises polyethylene oxide alone or in combination with at least one water-soluble polymer.

4. The process of claim 1, wherein said adjusting is adjusting the length of said film, or the width of said film, or the weight of said film.

5. The process of claim 1, wherein said adjusting is adjusting the length and width of said film.

6. The process of claim 1, wherein said adjusting is adjusting the thickness, or adjusting the weight of said film by applying an additional coating to said film.

7. The process of claim 1, wherein said analyzing is performed online or offline.

8. The process of claim 1, wherein said analyzing is performed online and followed by adjusting the thickness.

9. The process of claim 1, wherein said analyzing is performed offline and followed by adjusting the thickness, or the length or the width.

10. The process of claim 1, wherein said analyzing is performed offline and followed by adjusting the length and width.

11. The process of claim 1, wherein said active component is a pharmaceutical or biological active.

12. The process of claim 1, wherein said adjusting is cutting, or coating, or changing coating parameters.

13. The process of claim 1, wherein said adjusting is accomplished by a feedback control loop between the analyzer and the coating equipment.

14. The process of claim 1, wherein the film is analyzed and adjusted before drying or after drying.

15. The process of claim 1, wherein said preparing comprises:
(a) combining the polymer component, water and the active component to form a matrix with a uniform distribution of said components;
(b) forming a film from said matrix;
(c) providing a conveyor surface having top and bottom sides; and
(d) feeding said film onto said top side of said surface.

16. The process of claim 15, wherein said analyzing is performed online or offline.

17. The process of claim 15, wherein said analyzing is performed online or offline and followed by adjusting the thickness.

18. The process of claim 15, wherein said active component is a pharmaceutical or biological active.

19. The process of claim 15, wherein said adjusting is cutting or coating.

20. The process of claim 15, wherein said process further comprises a step (e) of analyzing said film to determine the amount of said active component per unit weight of said film and based on said amount of said component per unit weight of said film determining the dimension of the film necessary to deliver said predetermined dosage for said active component; and (f) adjusting said film into the dimension determined in step (e), wherein said process comprises drying said film, preferably drying said film after said step (e), or preferably drying said film before said step (e).

21. The process of claim 15, comprising packaging said film following said adjusting.

22. The process of claim 15, wherein said analyzing is performed at a predetermined time during said process, said analyzing preferably being performed by statistical sampling or being controlled by a computer programmed to adjust said film into a predetermined dimension and/or weight.

23. The process of claim 15, wherein said analyzing step comprises changing said predetermined dimension and/or weight to cut said film to a dimension and/or weight necessary to deliver said predetermined dosage based on said amount of said component per unit weight of said film.

24. The process of claim 1, wherein said preparing comprises:
a) preparing a continuous film comprising a polymer component and an active component uniformly dispersed in said polymer component, wherein said continuous film has a constant thickness;
wherein said analyzing comprises:
b) analyzing said continuous film online to determine the correct wet film thickness effective to produce a film dosage unit having said predetermined % of active per unit weight of said film dosage unit followed by the steps of:
c) preparing a continuous film having a wet thickness determined in step b):
d) drying said film; and
wherein said adjusting comprises:
e) cutting said film to produce said ingestible film dosage units.

25. The process of claim 1, wherein said preparing comprises:
a) preparing a continuous film comprising a polymer component and an active component uniformly dispersed in said polymer component;
b) drying said film;
wherein said analyzing comprises:
c) analyzing said dried film off line to determine the % of active per unit weight of said film;
d) using information obtained in said c) to determine the width and length of the film effective to produce a film having said predetermined dosage; and
wherein said adjusting comprises:
e) cutting said film to the width and length determined in said step d) to produce ingestible film dosage units.

## Patentansprüche

1. Verfahren zur Herstellung eines einnehmbaren Films, der eine aktive Komponente umfasst, wobei der einnehmbare Film eine vorbestimmte Dosierung für die aktive Komponente aufweist, wobei das Verfahren umfasst:
a) Herstellen eines kontinuierlichen Films, umfassend eine Polymerkomponente und die aktive Komponente, die gleichmäßig in der Polymerkomponente dispergiert ist;
b) Analysieren des Films, um die Menge der aktiven Komponente pro Gewichtseinheit des Films zu bestimmen, und basierend auf der Menge der Komponente pro Gewichtseinheit des Films, um eine Abmessung des Films zu bestimmen, die notwendig ist, um die vorbestimmte Dosierung für die aktive Komponente abzugeben;
c) Einstellen der Abmessung des Films in die in Schritt (b) bestimmte Abmessung.

2. Verfahren nach Anspruch 1, wobei die aktive Komponente ausgewählt ist aus der Gruppe, bestehend aus: Nahrungsmitteln, pharmazeutischen Mitteln, kosmetischen Mitteln, Arzneimitteln; Medikamenten; Gegenmitteln; Impfstoffen; Antigenen oder Allergenen; Mundwasser-Komponenten; Aromen; Duftstoffen; Enzymen; Konservierungsmitteln; Süßungsmitteln; Farbstoffen; Gewürzen; Vitaminen; Kühlmitteln; Kribbelmitteln und Kombinationen davon.

3. Verfahren nach Anspruch 1, wobei die Polymerkomponente Polyethylenoxid einzeln oder in Kombination mit mindestens einem wasserlöslichen Polymer umfasst.

4. Verfahren nach Anspruch 1, wobei das Einstellen das Einstellen der Länge des Films oder der Breite des Films oder des Gewichts des Films ist.

5. Verfahren nach Anspruch 1, wobei das Einstellen das Einstellen der Länge und Breite des Films ist.

6. Verfahren nach Anspruch 1, wobei das Einstellen das Einstellen der Dicke oder das Einstellen des Gewichts des Films durch Aufbringen einer zusätzlichen Beschichtung auf den Film ist.

7. Verfahren nach Anspruch 1, wobei das Analysieren online oder offline durchgeführt wird.

8. Verfahren nach Anspruch 1, wobei das Analysieren online durchgeführt wird und anschließend die Dicke angepasst wird.

9. Verfahren nach Anspruch 1, wobei das Analysieren offline durchgeführt wird und anschließend die Dicke oder die Länge oder die Breite angepasst wird.

10. Verfahren nach Anspruch 1, wobei das Analysieren offline durchgeführt wird und anschließend die Länge und Breite angepasst wird.

11. Verfahren nach Anspruch 1, wobei die aktive Komponente ein pharmazeutischer oder biologischer Wirkstoff ist.

12. Verfahren nach Anspruch 1, wobei das Einstellen Schneiden oder Beschichten oder Ändern von Beschichtungsparametern ist.

13. Verfahren nach Anspruch 1, wobei das Einstellen durch einen Rückkopplungsregelkreis zwischen dem Analysator und der Beschichtungsvorrichtung durchgeführt wird.

14. Verfahren nach Anspruch 1, wobei der Film vor dem Trocknen oder nach dem Trocknen analysiert und eingestellt wird.

15. Verfahren nach Anspruch 1, wobei die Herstellung umfasst:
(a) Kombinieren der Polymerkomponente, des Wassers und der aktiven Komponente zur Bildung einer Matrix mit einer gleichmäßigen Verteilung der Komponenten;
(b) Bilden eines Films aus der Matrix;
(c) Bereitstellen einer Förderfläche mit Ober- und Unterseite; und
(d) Zuführen des Films auf die Oberseite der Oberfläche.

16. Verfahren nach Anspruch 15, wobei das Analysieren online oder offline durchgeführt wird.

17. Verfahren nach Anspruch 15, wobei das Analysieren online oder offline durchgeführt wird und von der Einstellung der Dicke gefolgt wird.

18. Verfahren nach Anspruch 15, wobei die aktive Komponente ein pharmazeutischer oder biologischer Wirkstoff ist.

19. Verfahren nach Anspruch 15, wobei das Einstellen Schneiden oder Beschichten ist.

20. Verfahren nach Anspruch 15, wobei das Verfahren weiter einen Schritt (e) zum Analysieren des Films umfasst, um die Menge der aktiven Komponente pro Gewichtseinheit des Films zu bestimmen, und basierend auf der Menge der Komponente pro Gewichtseinheit des Films, um die Abmessung des Films zu bestimmen, die notwendig ist, um die vorbestimmte Dosierung für den aktiven Bestandteil abzugeben; und (f) Einstellen des Films in die in Schritt (e) bestimmte Abmessung, wobei das Verfahren das Trocknen des Films, vorzugsweise Trocknen des Films nach dem Schritt (e), oder vorzugsweise Trocknen des Films vor dem Schritt (e) umfasst.

21. Verfahren nach Anspruch 15, umfassend das Verpacken des Films nach dem Einstellen.

22. Verfahren nach Anspruch 15, wobei das Analysieren zu einem vorbestimmten Zeitpunkt während des Verfahrens durchgeführt wird, wobei das Analysieren vorzugsweise durch statistische Probenahme durchgeführt wird oder von einem Computer gesteuert wird, der programmiert ist, um den Film zu einer vorbestimmten Abmessung und/oder einem vorbestimmten Gewicht einzustellen.

23. Verfahren nach Anspruch 15, wobei der Analyseschritt das Ändern der vorbestimmten Abmessung und/oder des vorbestimmten Gewichts umfasst, um den Film auf eine Abmessung und/oder ein Gewicht zu schneiden, die notwendig ist, um die vorbestimmte Dosierung basierend auf der Menge der Komponente pro Gewichtseinheit des Films abzugeben.

24. Verfahren nach Anspruch 1, wobei die Herstellung umfasst:
a) Herstellen eines kontinuierlichen Films, umfassend eine Polymerkomponente und eine aktive Komponente, die gleichmäßig in der Polymerkomponente dispergiert ist, wobei der kontinuierliche Film eine konstante Dicke aufweist;
wobei das Analysieren umfasst:
b) Analysieren des kontinuierlichen Films online, um die richtige Nassfilmdicke zu bestimmen, die wirksam ist, um eine Filmdosiereinheit mit dem vorbestimmten Prozentsatz an Wirkstoff pro Gewichtseinheit der Filmdosiereinheit zu erzeugen, gefolgt von den Schritten von:
c) Herstellen eines kontinuierlichen Films mit einer in Schritt b) bestimmten Nassdicke:
d) Trocknen des Films; und
wobei die Einstellung umfasst:
e) Schneiden des Films, um die einnehmbaren Filmdosiereinheiten herzustellen.

25. Verfahren nach Anspruch 1, wobei die Herstellung umfasst:
a) Herstellen eines kontinuierlichen Films, umfassend eine Polymerkomponente und eine aktive Komponente, die gleichmäßig in der Polymerkomponente dispergiert ist;
b) Trocknen des Films;
wobei das Analysieren umfasst:
c) Analysieren des getrockneten Films offline, um den Prozentsatz an Wirkstoff pro Gewichtseinheit des Films zu bestimmen;
d) unter Verwendung der in dem c) erhaltenen Informationen, um die Breite und Länge des Films zu bestimmen, der wirksam ist, um einen Film mit der vorbestimmten Dosierung herzustellen; und
wobei die Einstellung umfasst:
e) Schneiden des Films auf die in dem Schritt d) bestimmte Breite und Länge, um einnehmbare Filmdosiereinheiten herzustellen.

## Revendications

1. Un procédé de production d'un film pouvant être ingéré comprenant un composant actif, ledit film pouvant être ingéré ayant un dosage prédéterminé dudit composant actif, ledit procédé comprenant les étapes de:
a) préparer un film continu comprenant un composant polymère et ledit composant actif dispersé uniformément dans ledit composant polymère;
b) analyser ledit film pour déterminer la quantité dudit composant actif par unité de poids dudit film et, sur la base de ladite quantité dudit composant par unité de poids dudit film, déterminer une dimension du film nécessaire pour délivrer ledit dosage prédéterminé pour ledit composant actif;
c) ajuster ladite dimension du film à la dimension déterminée à l'étape (b).

2. Le procédé selon la revendication 1, dans lequel ledit composant actif est choisi dans le groupe constitué par: des produits alimentaires, des agents pharmaceutiques, des agents cosmétiques, des médicaments; des antidotes; des vaccins; des antigènes ou des allergènes; des composants de bain de bouche; des saveurs; des parfums; des enzymes; des conservateurs; des agents édulcorants; des colorants; des épices; des vitamines; des agents de refroidissement; des agents de picotements, et leurs combinaisons.

3. Le procédé selon la revendication 1, dans lequel ledit composant polymère comprend de l'oxyde de polyéthylène seul ou en combinaison avec au moins un polymère soluble dans l'eau.

4. Le procédé selon la revendication 1, dans lequel ledit ajustement est un ajustement de la longueur dudit film, de la largeur dudit film ou du poids dudit film.

5. Le procédé selon la revendication 1, dans lequel ledit ajustement est un ajustement de la longueur et de la largeur dudit film.

6. Le procédé selon la revendication 1, dans lequel ledit ajustement est un ajustement de l'épaisseur ou un ajustement du poids dudit film en appliquant un revêtement supplémentaire sur ledit film.

7. Le procédé selon la revendication 1, dans lequel ladite analyse est effectuée en ligne ou hors ligne.

8. Le procédé selon la revendication 1, dans lequel ladite analyse est effectuée en ligne et est suivie d'un ajustement de l'épaisseur.

9. Le procédé selon la revendication 1, dans lequel ladite analyse est effectuée hors ligne et est suivie d'un ajustement de l'épaisseur, de la longueur ou de la largeur.

10. Le procédé selon la revendication 1, dans lequel ladite analyse est effectuée hors ligne et est suivie d'un ajustement de la longueur et de la largeur.

11. Le procédé selon la revendication 1, dans lequel ledit composant actif est un principe actif pharmaceutique ou biologique.

12. Le procédé selon la revendication 1, dans lequel ledit ajustement est une coupe, un revêtement ou une modification de paramètres de revêtement.

13. Le procédé selon la revendication 1, dans lequel ledit ajustement est réalisé par une boucle de contrôle de rétroaction entre l'analyseur et l'équipement de revêtement.

14. Le procédé selon la revendication 1, dans lequel le film est analysé et ajusté avant séchage ou après séchage.

15. Le procédé selon la revendication 1, dans lequel ladite préparation comprend les étapes de :
a) combiner le composant polymère, de l'eau et le composant actif pour former une matrice avec une distribution uniforme desdits composants;
b) former un film à partir de ladite matrice;
c) fournir une surface de convoyage ayant des côtés supérieur et inférieur; et
d) alimenter ledit film sur ledit côté supérieur de ladite surface.

16. Le procédé selon la revendication 15, dans lequel ladite analyse est effectuée en ligne ou hors ligne.

17. Le procédé selon la revendication 15, dans lequel ladite analyse est effectuée en ligne ou hors ligne et est suivie d'un ajustement de l'épaisseur.

18. Le procédé selon la revendication 15, dans lequel ledit composant actif est un principe actif pharmaceutique ou biologique.

19. Le procédé selon la revendication 15, dans lequel ledit ajustement est une coupe ou un revêtement.

20. Le procédé selon la revendication 15, dans lequel ledit procédé comprend en outre une étape (e) d'analyser ledit film pour déterminer la quantité dudit composant actif par unité de poids dudit film et, sur la base de ladite quantité dudit composant par unité de poids dudit film, déterminer la dimension du film nécessaire pour délivrer ledit dosage prédéterminé pour ledit composant actif; et une étape (f) d'ajustement dudit film à la dimension déterminée à l'étape (e), dans lequel ledit procédé comprend l'étape de, de préférence, sécher ledit film, sécher ledit film après ladite étape (e), ou, de préférence, sécher ledit film avant ladite étape (e).

21. Le procédé selon la revendication 15, comprenant le conditionnement dudit film après ledit ajustement.

22. Le procédé selon la revendication 15, dans lequel ladite analyse est effectuée à un moment prédéterminé pendant ledit procédé, ladite analyse étant de préférence effectuée par échantillonnage statistique ou sous commande d'un ordinateur programmé pour ajuster ledit film à une dimension et/ou un poids prédéterminés.

23. Le procédé selon la revendication 15, dans lequel ladite étape d'analyse comprend la modification desdites dimensions et/ou poids prédéterminés pour couper ledit film à une dimension et/ou poids nécessaires pour délivrer ledit dosage prédéterminé sur la base de ladite quantité dudit composant par unité de poids dudit film.

24. Le procédé selon la revendication 1, dans lequel ladite préparation comprend les étapes de :
a) préparer un film continu comprenant un composant polymère et un composant actif dispersé uniformément dans ledit composant polymère, dans lequel ledit film continu a une épaisseur constante;
dans lequel ladite analyse comprend les étapes de :
b) analyser ledit film continu en ligne pour déterminer l'épaisseur correcte du film humide permettant de produire une unité de dosage de film ayant ledit pourcentage prédéterminé de principe actif par unité de poids de ladite unité de dosage de film, analyse suivie des étapes de:
c) préparer un film continu ayant une épaisseur humide déterminée à l'étape b):
d) sécher ledit film; et
dans lequel ledit ajustement comprend l'étape de:
e) couper ledit film pour produire lesdites unités de dosage de film pouvant être ingéré.

25. Le procédé selon la revendication 1, dans lequel ladite préparation comprend les étapes de:
a) préparer un film continu comprenant un composant polymère et un composant actif dispersé uniformément dans ledit composant polymère;
b) sécher ledit film;
dans lequel ladite analyse comprend les étapes de :
c) analyser ledit film séché hors ligne pour déterminer le pourcentage de principe actif par unité de poids dudit film;
d) utiliser l'information obtenue à ladite étape c) pour déterminer la largeur et la longueur du film permettant de produire un film ayant ledit dosage prédéterminé; et
dans lequel ledit ajustement comprend l'étape de :
e) couper ledit film à la largeur et à la longueur déterminées à ladite étape d) pour produire des unités de dosage de film pouvant être ingéré.
